# EUROPEAN PATENT APPLICATION

(11) **EP 2 352 034 A1**
(43) Date of publication of application: **03.08.2011**
(21) Application number: 09846461.3
(22) Date of filing: 18.11.2009
(51) Int. Cl.: G01N 35/04, A61B 5/15, A61J 3/00, B65C 3/14

(54) **BLOOD COLLECTION TUBE STOCKER AND DEVICE FOR PREPARING BLOOD COLLECTION TUBES**

(30) Priority: 24.06.2009 JP 2009167245; 09.09.2009 JP 2009207765; 02.11.2009 JP 2009251674
(71) Applicant: Afc Limited, Osaka 553-0003 (JP)
(72) Inventor: YAMADA, Tadaichiro, Sakai-shi Osaka 587-0061 (JP)
(74) Representative: Mischung, Ralf
(86) International application number: PCT/JP2009/006204
(87) International publication number: WO 2010/150328

(57) **Abstract**

Provided is a blood collection tube preparing apparatus which is simple, downsized, portable, capable of being easily placed on a desk or the like, excellent in responding to an emergency such as a disaster, and capable of preparing various kinds of blood collection tubes depending on a blood collection instruction from a doctor. A blood collection tube holding member is arranged along one transferring belt and between a forward-side transferring belt and a backward-side transferring belt of the one transferring belt, and lower end surfaces of a cap of a blood collection tube is supported on upper end surfaces of the blood collection tube holding member and the forward-side transferring belt. The forward-side transferring belt applies a transferring force to the blood collection tube through the cap. Further, the members constituting a storage box are accommodated in one module, and the storage box is detachably mounted onto a mounting portion of an apparatus main body. Further, the storage box is in the form of module, and hence an operation in the case of failure or during maintenance is significantly facilitated.

## Description

### Technical Field

The present invention relates to a blood collection tube storage box and a blood collection tube preparing apparatus which perform an operation of preparing a blood collection tube in advance in a blood collection step as a pre-step of a blood collection test. The blood collection tube preparing apparatus adopts, in a form of module, the blood collection tube storage box which is allowed to be easily attached and detached, and in which the blood collection tube (including a test tube) is held in a suspended manner within an endless transferring belt and the blood collection tube is transferred by the transferring belt. The blood collection tube preparing apparatus can select various kinds of blood collection tubes from the storage box in the form of module depending on a blood collection instruction from a doctor, stick a label on which blood collection information such as a patient ID and a content of the blood collection instruction is printed, and receive the blood collection tube with the label into a tray for each patient. The blood collection tube preparing apparatus has a simple structure, and is downsized and excellent in portability.

### Background Art

In a blood test department of a hospital, a clinic, or another medical institution, a blood collection operator (such as nurse) collects and stores blood of a patient into a blood collection tube, and then transfers the blood collection tube to the test department. In a blood test, a wide variety of blood collection tubes are prepared correspondingly to blood test items. Normally, a plurality of kinds of tests are performed on one patient at the same time. Therefore, a plurality of kinds of blood collection tubes are automatically prepared for one patient by the blood collection tube preparing apparatus. Further, it is common to treat a large number of patients in a blood collection room, and a label, on which barcodes containing patient information, blood collection test information, and the like are printed, is stuck onto each of the blood collection tubes prepared by the blood collection tube preparing apparatus.
Further, when a patient comes to a reception desk to undergo blood collection, a reception ticket for blood collection, on which barcodes containing a reception number and the like are printed, is issued for the patient. The contents of the label and the reception ticket are read by an optical device such as a barcode reader for the purpose of preventing confusion of patients or blood collection tubes.

As a technology for achieving automation of blood collection operation as described above and for supporting the blood collection operation, there is known a technology disclosed in Patent Literature 1. The technology disclosed in Patent Literature 1 utilizes a belt driving device for an endless belt driven to rotate in a longitudinal direction (vertical direction). In the belt driving device, a belt width is set to be equal to or larger than a length dimension of the blood collection tube, and caterpillar-like partition plates protruding in a radially outward direction from a belt surface are provided. Further, one blood collection tube receiving portion (storage portion) is formed between adjacent partition plates on the belt surface, and a large number of blood collection tube receiving portions are formed on the one belt surface. In this case, the one belt driving device is set to receive blood collection tubes of the same kind. Further, storage box mechanisms each utilizing the belt driving device for receiving the blood collection tubes in the form of caterpillar are stacked and arranged in the vertical direction in a plurality of layers, and hence it is possible to handle a plurality of kinds of blood collection tubes.
Based on a blood collection order information from a doctor, a blood collection tube necessary for performing blood collection on a patient is taken out of the corresponding blood collection tube storage box mechanism. Using label printing/sticking means, information regarding the blood collection for the patient is printed on a label, and the printed label is stuck onto the taken-out blood collection tube. Then, the blood collection tube with the label is transferred to a blood collection tube collecting portion by discharging means, and is received into a tray for each patient by the blood collection tube collecting portion.

In the blood collection tube preparing apparatus as described above, the storage box mechanisms are stacked and arranged one above the other, and hence a dimension in a height direction is increased considerably. As a result, there is a problem in that the apparatus itself is increased in size.
Meanwhile, as a method of solving the above-mentioned conventional problem, there may be used, for a storage box, a technology of conveying a product similar in shape to the blood collection tube in a suspended manner as disclosed in Patent Literature 2 or Patent Literature 3. In the conveying technology, an annular flange portion (ring) provided in a vicinity of a mouth of a resin bottle or the like is used, and attachments are attached between two right and left conveying belts so as to allow the ring to be engaged and supported with respect to the conveying belts. Thus, the resin bottle or the like is conveyed in a suspended manner while the ring of the resin bottle or the like is engaged with the pair of opposing attachments.

### Citation List

### Patent Literature

Patent Literature 1: JP 2005-67660 A
Patent Literature 2: JP 2006-21854 A
Patent Literature 3: JP 2007-145493 A

### Summary of the Invention

### Technical Problems

However, in the technology disclosed in Patent Literature 2 or Patent Literature 3, a pair of the two conveying belts arranged in parallel to each other constitutes one conveying device, and hence two driving mechanisms for the conveying belts are necessary for the one conveying device.
Further, when the two conveying belts are arranged so as to be driven to rotate in a horizontal direction, a dimension obtained by adding diameters of two pulleys of the right and left conveying belts, thickness dimensions of the belts, dimensions of the attachments, and a width dimension of a product to be conveyed are indispensable. Therefore, an entire dimension in a width direction is increased considerably. As a result, in the case where the blood collection tube preparing apparatus is structured as an apparatus for automatically preparing a wide variety of blood collection tubes, it is impossible to avoid an increase in size of the entire apparatus. Therefore, there is a problem in that the technology disclosed in Patent Literature 2 or Patent Literature 3 is not applicable to the blood collection tube preparing apparatus as it is.

As described above, in the technology disclosed in Patent Literature 1, the receiving portions for the wide variety of blood collection tubes are stacked and arranged in the vertical direction, and hence there is a problem in that a height dimension in the vertical direction is increased. In the case where the technology disclosed in Patent Literature 2 or Patent Literature 3 is applied to a technology for the blood collection tube preparing apparatus as it is, a width dimension in the horizontal direction is increased. Accordingly, when the blood collection tube preparing apparatus is structured as an apparatus for handling the wide variety of blood collection tubes, there is a problem in that the apparatus itself is increased in size. Further, in the case where the technology disclosed in Patent Literature 2 or Patent Literature 3 is used as it is, there is a problem in that the number of the conveying mechanisms needs to be twice the number of the kinds of the blood collection tubes to be handled.
The above-mentioned problems of the size increase of the blood collection tube preparing apparatus lead to problems in that, when changing a layout, for example, it is impossible to easily move the blood collection tube preparing apparatus, and in that limitation is imposed in terms of a space particularly when applying the apparatus to a small-scale hospital. In addition, the problems of the size increase become one of factors that impede coping with such case requiring easy placement as in a disaster, and hence there is a disadvantage that emergency response is impossible.

Therefore, in view of the above-mentioned conventional problems, in order to eliminate the conventional problems, it is an object of the present invention to provide a blood collection tube storage box of a suspended type capable of being easily attached and detached in the form of module, and to provide a blood collection tube preparing apparatus of an all-in-one type using the blood collection tube storage box. The blood collection tube storage box is simple, downsized, portable, capable of being moved on a wagon to a bedside and of being easily mounted on a desk or the like only through being placed thereon, excellent in responding to an emergency such as a disaster, and capable of preparing various kinds of blood collection tubes depending on a blood collection instruction from a doctor.

### Solution to Problems

Therefore, in order to solve the above-mentioned problems, means of claim 1 adopted by the present invention is a blood collection tube storage box, including: an endless transferring belt which is looped around pulleys respectively provided on both end sides of the endless transferring belt, and which is driven to rotate in a horizontal direction; a blood collection tube holding member which is situated between a forward-side transferring belt and a backward-side transferring belt of the endless transferring belt, and which is arranged along the endless transferring belt, the forward-side transferring belt and the blood collection tube holding member respectively having upper end surfaces on which opposing lower end surfaces of a cap of a blood collection tube are placed; a discharging portion through which the blood collection tube falls down by an own weight of the blood collection tube to be discharged, at a position at which the blood collection tube passes a terminal end side of the blood collection tube holding member; a first stopper and a second stopper provided on the terminal end side of the blood collection tube holding member, the first stopper being capable of moving close to and away from a front surface of a cap of a leading blood collection tube, the second stopper having a fork-like shape and being capable of moving close to and away from a gap from above, the gap being formed between the cap of the leading blood collection tube and a cap of a second blood collection tube; two arms which are pivotally supported by the same shaft, and to which the first stopper and the second stopper are fixed on distal end sides of the two arms, respectively; and solenoids coupled on trailing end sides of the two arms, respectively, the endless transferring belt, the blood collection tube holding member, the discharging portion for the blood collection tube, the first stopper, the second stopper, the two arms, and the solenoids being accommodated in one casing, to thereby form a module.

Means of claim 2 adopted by the present invention is a blood collection tube storage box, including: an endless transferring belt which is looped around pulleys respectively provided on both end sides of the endless transferring belt, and which is driven to rotate in a horizontal direction; two blood collection tube support rails which are situated between a forward-side transferring belt and a backward-side transferring belt of the endless transferring belt and above an upper end of the endless transferring belt, and which respectively support opposing lower end surfaces of a cap of a blood collection tube in a suspended manner, the forward-side transferring belt having an inner flat surface brought into contact with a side surface of a tube main body of the blood collection tube supported by the two blood collection tube support rails in the suspended manner; a discharging portion through which the blood collection tube falls down by an own weight of the blood collection tube to be discharged, at a position at which the blood collection tube passes a terminal end side of the two blood collection tube support rails; a first stopper and a second stopper provided on the terminal end side of the two blood collection tube support rails, the first stopper being capable of moving close to and away from a front surface of a cap of a leading blood collection tube, the second stopper having a fork-like shape and being capable of moving close to and away from a gap from above, the gap being formed between the cap of the leading blood collection tube and a cap of a second blood collection tube; two arms which are pivotally supported by the same shaft, and to which the first stopper and the second stopper are fixed on distal end sides of the two arms, respectively; and solenoids coupled on trailing end sides of the two arms, respectively, the endless transferring belt, the two blood collection tube support rails, the discharging portion for the blood collection tube, the first stopper, the second stopper, the two arms, and the solenoids being accommodated in one casing, to thereby form a module.

Means of claim 3 adopted by the present invention is a blood collection tube preparing apparatus, including: a plurality of blood collection tube storage boxes, each of the plurality of blood collection tube storage boxes including: an endless transferring belt which is looped around pulleys respectively provided on both end sides of the endless transferring belt, and which is driven to rotate in a horizontal direction; a blood collection tube holding member which is situated between a forward-side transferring belt and a backward-side transferring belt of the endless transferring belt, and which is arranged along the endless transferring belt, the forward-side transferring belt and the blood collection tube holding member respectively having upper end surfaces on which opposing lower end surfaces of a cap of a blood collection tube are placed; a discharging portion through which the blood collection tube falls down by an own weight of the blood collection tube to be discharged, at a position at which the blood collection tube passes a terminal end side of the blood collection tube holding member; a first stopper and a second stopper provided on the terminal end side of the blood collection tube holding member, the first stopper being capable of moving close to and away from a front surface of a cap of a leading blood collection tube, the second stopper having a fork-like shape and being capable of moving close to and away from a gap from above, the gap being formed between the cap of the leading blood collection tube and a cap of a second blood collection tube; two arms which are pivotally supported by the same shaft, and to which the first stopper and the second stopper are fixed on distal end sides of the two arms, respectively; and solenoids coupled on trailing end sides of the two arms, respectively, the endless transferring belt, the blood collection tube holding member, the discharging portion for the blood collection tube, the first stopper, the second stopper, the two arms, and the solenoids being accommodated in a module serving as one casing, the plurality of blood collection tube storage boxes being detachably and respectively received, in a form of the modules, in a plurality of lanes aligned in parallel to each other; a conveying belt provided below a discharging section ranging over a region of the discharging portion of each of the plurality of blood collection tube storage boxes; label printing/sticking means provided on a terminal end side of the conveying belt; and a tray disposing portion provided in a vicinity of a delivery portion of the label printing/sticking means, for receiving the blood collection tube.

Means of claim 4 adopted by the present invention is a blood collection tube preparing apparatus, including: an endless transferring belt which is looped around pulleys respectively provided on both end sides of the endless transferring belt, and which is driven to rotate in a horizontal direction; two blood collection tube support rails which are situated between a forward-side transferring belt and a backward-side transferring belt of the endless transferring belt and above an upper end of the endless transferring belt, and which respectively support opposing lower end surfaces of a cap of a blood collection tube in a suspended manner, the forward-side transferring belt having an inner flat surface brought into contact with a side surface of a tube main body of the blood collection tube supported by the two blood collection tube support rails in the suspended manner; a discharging portion which is provided on a terminal end side of the two blood collection tube support rails, and through which the blood collection tube falls down by an own weight of the blood collection tube to be discharged; a conveying belt provided below the discharging portion, for conveying the blood collection tube; label printing/sticking means provided on a terminal end side of the conveying belt; and a tray disposing portion provided in a vicinity of a delivery portion of the label printing/sticking means, for receiving the blood collection tube.

Means of claim 5 adopted by the present invention is a blood collection tube preparing apparatus, including: a plurality of blood collection tube storage boxes, each of the plurality of blood collection tube storage boxes including: an endless transferring belt which is looped around pulleys respectively provided on both end sides of the endless transferring belt, and which is driven to rotate in a horizontal direction; two blood collection tube support rails which are situated between a forward-side transferring belt and a backward-side transferring belt of the endless transferring belt and above an upper end of the endless transferring belt, and which respectively support opposing lower end surfaces of a cap of a blood collection tube in a suspended manner, the forward-side transferring belt having an inner flat surface brought into contact with a side surface of a tube main body of the blood collection tube supported by the two blood collection tube support rails in the suspended manner; a discharging portion through which the blood collection tube falls down by an own weight of the blood collection tube to be discharged, at a position at which the blood collection tube passes a terminal end side of the two blood collection tube support rails; a first stopper and a second stopper provided on the terminal end side of the two blood collection tube support rails, the first stopper being capable of moving close to and away from a front surface of a cap of a leading blood collection tube, the second stopper having a fork-like shape and being capable of moving close to and away from a gap from above, the gap being formed between the cap of the leading blood collection tube and a cap of a second blood collection tube; two arms which are pivotally supported by the same shaft, and to which the first stopper and the second stopper are fixed on distal end sides of the two arms, respectively; and solenoids coupled on trailing end sides of the two arms, respectively, the endless transferring belt, the two blood collection tube support rails, the discharging portion for the blood collection tube, the first stopper, the second stopper, the two arms, and the solenoids being accommodated in a module serving as one casing, the plurality of blood collection tube storage boxes being detachably and respectively received, in a form of the modules, in a plurality of lanes aligned in parallel to each other; a conveying belt provided below a discharging section ranging over a region of the discharging portion of each of the plurality of blood collection tube storage boxes; label printing/sticking means provided on a terminal end side of the conveying belt; and a tray disposing portion provided in a vicinity of a delivery portion of the label printing/sticking means, for receiving the blood collection tube.

Means of claim 6 adopted by the present invention is a blood collection tube preparing apparatus according to any one of claims 3 to 5, further including a sensor provided on the tray disposing portion, for detecting presence/absence of a tray, in which, when receiving, after receiving the blood collection tube for one patient into the tray, a blood collection tube for a next patient into another tray, each component constituting the blood collection tube preparing apparatus is prevented from starting to operate unless the sensor detects the another tray after detecting that the tray for the one patient is removed.

Means of claim 7 adopted by the present invention is a blood collection tube preparing apparatus according to any one of claims 3 to 6, further including an opening portion which is formed in a vicinity of the discharging portion of one of the plurality of blood collection tube storage boxes situated on a most upstream side of the conveying belt, and which allows the blood collection tube to be manually inserted onto the conveying belt through the opening portion.

Means of claim 8 adopted by the present invention is a blood collection tube preparing apparatus according to any one of claims 3 to 7, further including a separate-type versatile printer built in at a position in a vicinity of the label printing/sticking means and facing the tray disposing portion.

Means of claim 9 adopted by the present invention is a blood collection tube preparing apparatus according to any one of claims 3 to 8, further including a board computer built in a blood collection tube preparing apparatus main body, the board computer being capable of communicating to an upper computer and being provided with various user interfaces.

Means of claim 10 adopted by the present invention is a blood collection tube preparing apparatus according to any one of claims 3 to 9, in which the label printing/sticking means is slidably attached, as a printer unit, to the blood collection tube preparing apparatus main body so as to be capable of being pulled out of the blood collection tube preparing apparatus main body and replaced by a commercially available product.

Means of claim 11 adopted by the present invention is a blood collection tube preparing apparatus according to any one of claims 3 to 10, further including a start switch for the blood collection tube preparing apparatus, which is turned ON and OFF through remote control.

Means of claim 12 adopted by the present invention is a blood collection tube preparing apparatus according to any one of claims 3 to 11, further including a setting state display portion provided on an outer surface of the blood collection tube preparing apparatus main body, for displaying a setting state of the blood collection tube preparing apparatus and displaying guidance for changing settings through interlocking with each operation switch.

Means of claim 13 adopted by the present invention is a blood collection tube preparing apparatus according to any one of claims 3 to 12, further including: a central processing unit (CPU) which is built in or externally connected to the blood collection tube preparing apparatus, and on which a software program for controlling the blood collection tube preparing apparatus is installed; and a touch panel display provided on the outer surface of the blood collection tube preparing apparatus main body, for functioning both as an input portion and a monitor for the CPU.

### Advantageous Effects of Invention

According to the invention of claim 1, the blood collection tube holding member is provided between the forward-side transferring belt and the backward-side transferring belt of the one endless transferring belt driven to rotate in the horizontal direction. The blood collection tube holding member is a guide frame formed of a rail-like or plate-like member or the like having a smoothed surface, and supports one of the lower end surfaces of the cap of the blood collection tube. The other of the opposing lower end surfaces of the cap is supported on the upper end surface of the forward-side transferring belt. Therefore, the blood collection tube is moved while sliding on the blood collection tube holding member and the forward-side transferring belt under a state in which the lower end surfaces of the cap are respectively supported on the upper end surfaces of the blood collection tube holding member and the forward-side transferring belt. The blood collection tube is transferred through bringing the upper end surface of the forward-side transferring belt and the lower end surface of the cap of the blood collection tube into contact with each other.
On the terminal end side of the blood collection tube holding member, the blood collection tube is disengaged from the blood collection tube holding member to be cantilever-supported on the upper end surface of the forward-side transferring belt. Then, the cap (head) side is inclined toward the blood collection tube holding member disengaged from the blood collection tube so that the blood collection tube falls down. Thereafter, the blood collection tube is discharged.

As described above, according to the invention of claim 1, between the forward-side transferring belt and the backward-side transferring belt of the one transferring belt, the storage box of a suspended type for supporting the lower end surfaces of the cap of the blood collection tube is formed. Therefore, the conveying device for the blood collection tube in the storage box can be structured as one conveying device for one kind of blood collection tube, and hence the number of conveying devices can be set to be requisite minimum. Further, the blood collection tube storage box is formed between the forward-side transferring belt and the backward-side transferring belt of the one transferring belt, and hence one storage box has a width of only a few centimeters. Thus, even in the case where a plurality of kinds of storage boxes are aligned with each other in order to correspond to various kinds of blood collection tubes, the entire width dimension can be advantageously and remarkably reduced in comparison with the prior art.

Further, according to the invention of claim 1, there is adopted a mechanism for discharging the leading blood collection tube through isolating the leading blood collection tube from the other blood collection tubes, and the first stopper and the second stopper are provided. The first stopper is capable of moving close to and away from the front surface side of the cap of the leading blood collection tube so as to abut against the same. The second stopper is capable of moving close to and away from the gap formed between the caps of the leading and second blood collection tubes.

The first stopper abuts against the front surface side of the cap of the leading blood collection tube, and the second stopper enters the gap formed between the caps of the leading and second blood collection tubes. Under this state, the first stopper is operated to move away from the leading blood collection tube, and to disengage from the front surface side of the cap of the leading blood collection tube. Then, the leading blood collection tube is further moved ahead through being transferred while the upper end surface of the forward-side transferring belt and the lower end surface of the cap are held in contact with each other. After a while, the terminal end side of the blood collection tube holding member is disengaged. Therefore, the leading blood collection tube assumes such a posture that the cap side thereof is inclined toward the side on which the blood collection tube holding member is disengaged. As a result, the upper end surface of the forward-side transferring belt on the other side is also disengaged so that the leading blood collection tube falls down by its own weight to be discharged. The second and subsequent blood collection tubes remain stopped owing to the second stopper. After the leading blood collection tube is discharged, the first stopper is operated to return. When the second stopper is operated to move away from the gap, all of the remaining blood collection tubes are pushed to move ahead by a length of one blood collection tube, and the first stopper stops the movement of the remaining blood collection tubes. Then, the second stopper is operated to return to the original state.

In addition, according to the invention of claim 1, each of the first stopper and the second stopper is fixed to, for example, one end side of the arm, a middle portion of the arm is swingably and pivotally supported, and the solenoid (distal end of a rod of the solenoid) is coupled to the other end side of the arm. Therefore, through performing control to turn the solenoid ON and OFF, the rod of the solenoid is operated to protrude and retract with respect to a main body case, and each of the first stopper and the second stopper fixed on the one end side of the arm is operated to be capable of moving close to and away from the cap of the blood collection tube.
As described above, the first stopper and the second stopper are controlled and operated to move close to and away from the cap of the blood collection tube, and thus it is possible to discharge the leading blood collection tube through insolating the leading blood collection tube from the other blood collection tubes.

Further, according to the invention of claim 1, the conveying belt for conveying the blood collection tube in a laid posture is arranged below the terminal end side of the blood collection tube holding member. As described in the invention of claim 2, the conveying belt sweeps over the bottom of the blood collection tube falling down to be supplied while being inclined from the terminal end side of the blood collection tube holding member, and conveys the blood collection tube in the laid posture. Therefore, the blood collection tube is conveyed in a state in which the bottom side is always directed forward in the conveying direction, and thus it is possible to omit time and effort to align the orientations of the blood collection tubes in the next label sticking step.

Still further, according to the invention of claim 1, respective components such as the transferring belt, the blood collection tube holding member, the discharging portion for the blood collection tube, the first stopper, the second stopper, the two arms, and the solenoids are accommodated in one casing, to thereby form a module. With formation of the module as described above, if modules corresponding to the kinds of the blood collection tube are prepared, even when the tube kind of the blood collection tube is to be changed, it is only necessary to replace the module, and thus extremely easy and prompt handling operation is possible. In addition, in case of a failure, the blood collection tube storage box can be send back for repair on a module basis, and the repair can be easily performed even at remote locations.

According to the invention of claim 2, above the transferring belt, the lower end surfaces of the cap of the blood collection tube are respectively supported by the two blood collection tube support rails. Therefore, it is possible to store a large number of blood collection tubes between the two support rails only by inserting the blood collection tubes therebetween. Further, the blood collection tube is transferred through bringing the transferring belt into contact with a side surface of the blood collection tube main body. With the above-mentioned storing method and conveying method for the blood collection tube, the transferring belt and the cap of the blood collection tube are not brought into contact with each other, and hence there is no fear that the blood collection tube slips down due to distortion of the transferring belt. Isolation of the blood collection tube is performed through alternately causing the first stopper and the second stopper to operate, the first stopper being capable of moving close to and away from the front surface of the cap of the leading blood collection tube, the second stopper having a fork-like shape and being capable of moving close to and away from the gap from above, the gap being formed between the caps of the leading and second blood collection tubes.
Further, the blood collection tube storage box is in the form of module in which respective operation mechanisms are accommodated in one casing. In case of a failure, the blood collection tube storage box can be sent back for repair after detaching only the module, which is remarkably convenient for a customer at remote locations.

The blood collection tube preparing apparatus according to the invention of claim 3 uses the blood collection tube storage box according to the invention of claim 1, and conveys the blood collection tube discharged from the storage box through being isolated from the other blood collection tubes up to a position of the label printing/sticking means in a state in which the blood collection tube is laid so that the bottom side of the blood collection tube is directed forward. The label printing/sticking means obtains blood collection information of the blood collection patient from, for example, the upper system or a doctor's personal computer (PC) terminal, performs printing on a label, and sticks the printed label onto an outer peripheral surface of the blood collection tube. After being subjected to label sticking, the blood collection tube is received into the tray for one and the same patient.
With the above-mentioned blood collection tube preparing apparatus using the blood collection tube storage box, even when the blood collection tube preparing apparatus is structured to be capable of handling various kinds of blood collection tubes, the blood collection tube preparing apparatus has an extremely small size, and has an excellent characteristic in terms of lightweight and downsizing of the entire apparatus.

Therefore, the blood collection tube preparing apparatus according to the invention of claim 3 is compact and portable, and is easily moved. Accordingly, in the case of a disaster or the like, it is extremely easy to carry and place the blood collection tube preparing apparatus in an emergency facility or the like on the site. It is possible to capture information regarding blood collection performed on the patient that a doctor examines through connection to the computer terminal such as a laptop PC that the doctor uses, and to take the blood collection tube necessary for the blood collection out of the storage box selectively. Further, it is possible to receive the blood collection tube into the tray for each patient after sticking the printed label having the blood collection information of the patient onto the taken-out blood collection tube, and to automatically prepare for the blood collection. Further, there is an advantage that, for example, the blood collection tube preparing apparatus can be introduced also in a private hospital or the like. In addition, in in-patients wards, it is possible to bring the blood collection tube preparing apparatus on a movable wagon to the patient's bedside in each ward, to automatically prepare the blood collection tube of the apparatus, and to perform blood collection.

Moreover, the blood collection tube preparing apparatus according to the invention of claim 3 uses the blood collection tube storage box in the form of module. Therefore, according to the invention of claim 3, a synergistic effect can be obtained due to the form of module described in claim 1, and hence it is possible not only to facilitate an operation of, for example, changing the tube kind, but also to send back for repair on a module basis for maintenance. During the maintenance, it is possible to perform a blood collection preparing operation using another module of the same tube kind, and it is unnecessary to stop the blood collection preparing operation for the maintenance and repair.

In the blood collection tube preparing apparatus according to the invention of claim 4, after the blood collection tube is isolated from the other blood collection tubes with use of the blood collection tube storage box of a two-rail supporting method, the blood collection tube is conveyed to the label printing/sticking means, and necessary information, such as patient information and test information, is printed on a label. Then, the label is stuck onto the outer peripheral side surface of the blood collection tube, and the blood collection tube is put onto the tray portion.
When the blood collection tube storage boxes are provided in a plurality of rows, it is possible to prepare a plurality of kinds of blood collection tubes. Even in this case, a width of one blood collection tube storage box is about 30 to 40 mm, and a maximum width of the entire apparatus is extremely reduced. As a result, it is possible to achieve downsizing.

In the blood collection tube preparing apparatus according to the invention of claim 5, a plurality of kinds of blood collection tubes are selected using the blood collection tube storage boxes in the form of modules for conveying the blood collection tubes suspended and supported by the two blood collection tube support rails described in claim 2. Further, the printed label having necessary information, such as patient information and test information, is stuck onto each of the blood collection tubes so that the blood collection tubes for one patient are prepared. Other actions and effects are the same as those of the invention of claim 3 and the invention of claim 4.

According to the invention of claim 6, in the tray disposing portion, the sensor for detecting presence/absence of the tray is arranged. Further, when receiving, after receiving the blood collection tube for one patient into the tray, a blood collection tube for the next patient into another tray, each component is prevented from starting to operate unless the sensor detects the another tray after detecting that the tray for the one patient is removed. In this way, it is possible to reliably receive the blood collection tube for each patient, and to prevent confusion with the blood collection tube for another patient.

According to the invention of claim 7, on the upstream side of the conveying belt for supplying, to the label printing/sticking means, the blood collection tube discharged from each of the blood collection tube storage boxes, the opening portion through which the blood collection tube can be manually put onto the conveying belt is provided. This is provided to treat the patient requiring a rarely-performed blood collection test or a special blood collection test. In each of the blood collection tube storage boxes, the blood collection tubes of a frequently-used tube kind are stored. In the case of the rarely-performed blood collection test or the special blood collection test, individual treatment is necessary. Thus, according to the invention of claim 7, the opening portion is provided to allow an operator to manually supply the blood collection tube used for the rarely-performed blood collection test or the special blood collection test, and the blood collection tube is supplied to the label printing/sticking means.

According to the invention of claim 8, the separate-type versatile printer is built in the blood collection tube preparing apparatus main body. This is used, for example, to print a blood collection instruction. Further, in a hospital ward or the like, in the case where it is uncertain whether or not the patient is in a condition of allowing blood collection, only printing of necessary information, such as patient information and test information, is performed on the label by the separate-type printer without sticking the label onto the blood collection tube. With this structure, it is possible to perform blood collection directly by the patient's bedside depending on the condition of the patient, and to stick the manual sticking label onto the blood collection tube after performing the blood collection. When the patient is in a condition of not allowing blood collection, the manual sticking label may be left in the tray.

According to the invention of claim 9, the built-in computer board capable of connecting to an upper medical computer or a host computer via a LAN or of connecting to another user interface is provided. With this structure, it is also possible to obtain patient information, test information, and the like from the upper computer. Further, it is possible to structure a blood collection tube preparing apparatus of an all-in-one type.

According to the invention of claim 10, as the printer unit, the printing/sticking means is slidably attached to the apparatus main body. Therefore, for example, when the printer unit fails, the printer unit can be replaced with a substitute to maintain a function of the apparatus, and the failed printer unit can be sent back for repair, which is convenient in terms of maintenance. When the separate-type printer is mounted, similarly to the printer unit, the separate-type printer can be replaced and repaired, or sent back for repair.

According to the invention of claim 11, the start switch for the blood collection tube preparing apparatus is turned ON and OFF through remote control. For example, in a hospital or the like, upon start or termination of the operation for blood collection test, a chief technology officer or an operator of a blood collection laboratory must turn ON or OFF the start switch for the blood collection tube preparing apparatus and turn ON or OFF a start switch for the CPU for controlling the blood collection tube preparing apparatus. However, as described in the invention of claim 11, when the start switch can be turned ON and OFF through remote control, an upper operator can remote control start or shutdown of the CPU and the blood collection tube preparing apparatus via the LAN in the hospital, which is remarkably convenient.
Further, the start switch for the blood collection tube preparing apparatus can be turned ON and OFF through control from the input portion for the CPU. In this case, in the CPU, power sources of the CPU and the blood collection tube preparing apparatus can be simultaneously turned ON and OFF on the CPU side, which is convenient.

According to the invention of claim 12, the setting state display portion is provided on the outer surface of the apparatus main body. The setting state display portion displays the setting state of the apparatus and displays the guidance for changing settings through interlocking with the function operation switch for each component of the apparatus main body. With this structure, it is possible to visually confirm the current setting state of the apparatus, and to display the contents to be changed and the like in the case of setting change.
Therefore, an operator can reliably confirm the setting state without any connection to an external addition device, and can advantageously perform the setting change.

The blood collection tube preparing apparatus according to the invention of claim 13 includes the built-in or externally-connected CPU, and the software program for controlling the blood collection tube preparing apparatus is installed on the CPU. Further, the touch panel display is provided on the outer surface of the apparatus main body, for functioning both as the input portion and the monitor for the CPU. It is possible to display, on the touch panel display, patient information and test information obtained from a hospital information system (HIS) and a laboratory information system (LIS) serving as upper systems for the blood collection tube preparing apparatus. The patient information includes a patient ID, information that the patient is infected with new influenza, human immunodeficiency virus (HIV), or the like, and information that the patient is physically-disabled. With this, an operator can recognize who the patient is, and can individually take measures through applying an identifying mark onto the tray for the patient in order to indicate that the patient is infected with particular pathogens, or through supplying the tray to the blood collection site separately from a tray for a normal patient. Based on the above-mentioned information, a blood collection operator can take measures corresponding to each patient, and thus safe blood collection operation can be performed.

As the test information, for example, there are displayed items regarding the blood collection test, the items including a kind of test such as biochemical test, immunological test, blood glucose test, hematological test, or clotting test, a patient name, a container name of a blood collection tube, a barcode as an arbitrary ID relating to patient, and a blood collection volume. Thus, an operator can visually confirm whether or not the blood collection tube for the test is set in the blood collection tube preparing apparatus. When the blood collection tube is not set therein, the operator can prepare the blood collection tube to be manually inserted.

Further, the touch panel display can display a touch key for performing function operation on the CPU, and also has a function as the input portion for the CPU. Therefore, in the case of the built-in CPU, an operator can control the blood collection tube preparing apparatus through the touch key displayed on the touch panel display. Further, in the case of the externally-connected CPU, an operator can input from a keyboard of the externally-connected CPU or input through the touch key displayed on the touch panel display. Through utilizing the touch panel display, without operating a plurality of devices and moving for the operating, an operator can perform all operations in the blood collection tube preparing apparatus. Thus, the blood collection tube preparing apparatus is useful, and reduces a burden on the operator.

### Brief Description of Drawings

[FIG. 1] A plan view illustrating an entire structure of a blood collection tube preparing apparatus according to an embodiment of the present invention.
[FIG. 2] A front view illustrating the entire structure of the blood collection tube preparing apparatus according to the embodiment of the present invention.
[FIG. 3] A back view illustrating the entire structure of the blood collection tube preparing apparatus according to the embodiment of the present invention.
[FIG. 4] A left-hand side view illustrating the entire structure of the blood collection tube preparing apparatus according to the embodiment of the present invention.
[FIG. 5] A plan view illustrating a basic structure of a blood collection tube storage box according to the embodiment of the present invention.
[FIG. 6] A side view illustrating an entire structure of the blood collection tube storage box according to the embodiment of the present invention.
[FIG. 7] A sectional view of the blood collection tube storage box according to the embodiment of the present invention taken along the line A-A of FIG. 5.
[FIG. 8] A plan view illustrating a first stopper and a second stopper according to the embodiment of the present invention.
[FIG. 9] A vertical sectional front view illustrating the blood collection tube preparing apparatus according to the embodiment of the present invention.
[FIG. 10] A side view illustrating positional relation among members constituting a label sticking device according to the embodiment of the present invention.
[FIG. 11] A plan view illustrating an entire structure of a blood collection tube preparing apparatus according to a second embodiment of the present invention.
[FIG. 12] A front view illustrating the entire structure of the blood collection tube preparing apparatus according to the second embodiment of the present invention.
[FIG. 13] A plan view illustrating a basic structure of a blood collection tube storage box according to the second embodiment of the present invention.
[FIG. 14] A side view illustrating an entire structure of the blood collection tube storage box according to the second embodiment of the present invention.
[FIG. 15] A sectional view illustrating the blood collection tube storage box according to the second embodiment of the present invention taken along the line A-A of FIG. 13.
[FIG. 16] A side view illustrating a blood collection tube supporting mechanism and a transferring mechanism of the blood collection tube storage box according to the second embodiment of the present invention.

### Description of Embodiments

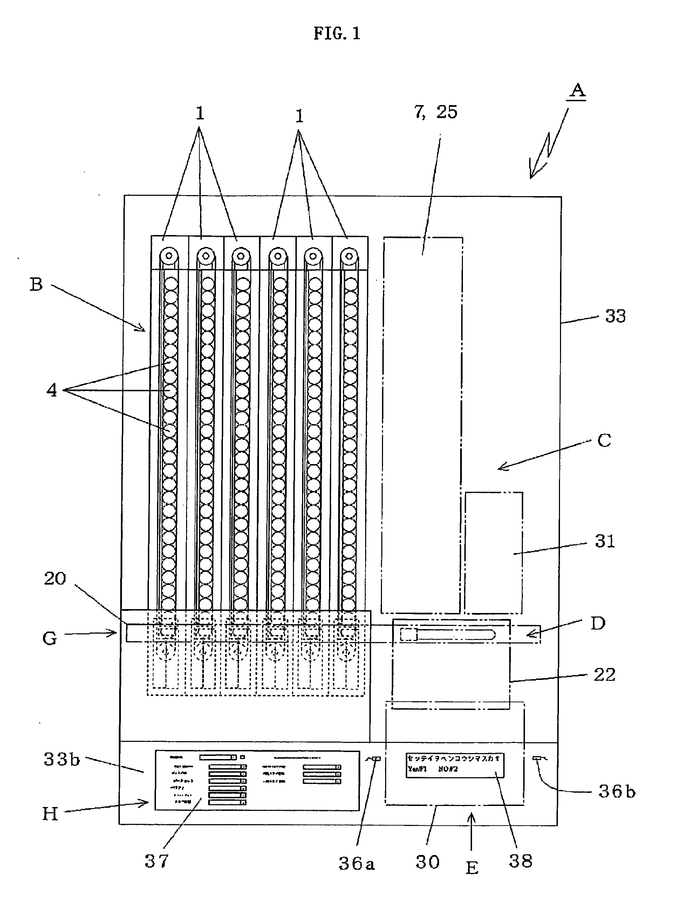

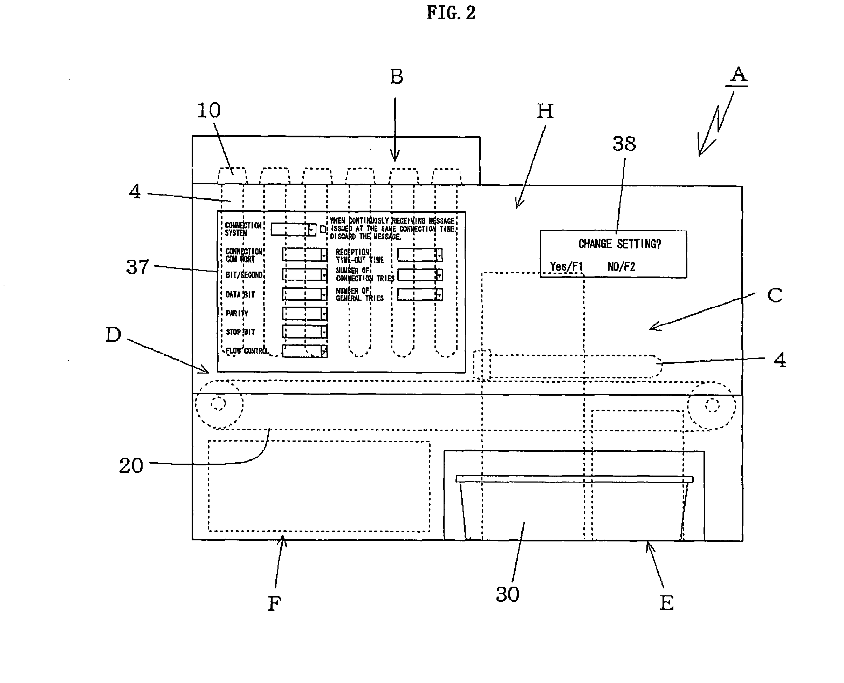

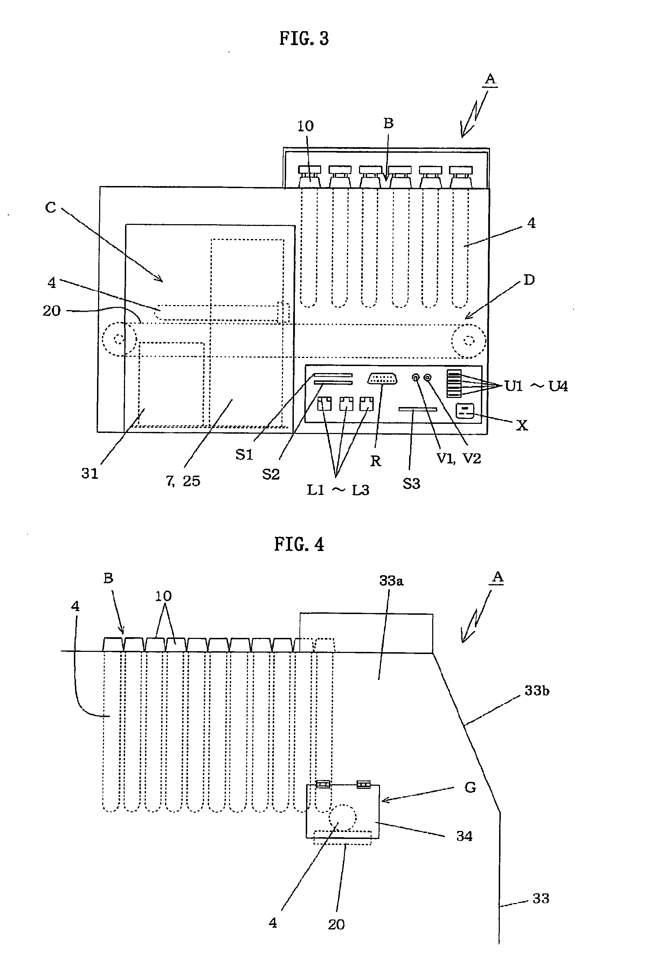

Hereinafter, a structure of the present invention is described as follows based on a first embodiment illustrated in FIGS. 1 to 10. First, a layout of equipment and mechanisms constituting a blood collection tube preparing apparatus A is described with reference to FIGS. 1 to 4. FIGS. 1 to 4 illustrate an entire structure of the blood collection tube preparing apparatus A. FIG. 1 is a plan view, FIG. 2 is a front view, FIG. 3 is a back view, and FIG. 4 is a left-hand side view. As illustrated in the plan view of FIG. 1, in the blood collection tube preparing apparatus A, a mounting portion B for a plurality of blood collection tube storage boxes 1 is provided to occupy almost a left half region. In this embodiment, six storage boxes 1 are placed in parallel with each other in the form of modules, and the modules of the blood collection tube storage boxes 1 are detachably mounted to the mounting portion on an apparatus main body 33 side. On a right side of the storage box mounting portion B and inside the apparatus main body, a placing portion C for label printing/sticking means is provided. Further, below a blood collection tube discharging portion of each of the storage boxes 1 on the storage box mounting portion B, there is provided a placing portion D for a conveying belt 20 which crosses the placing portion C for the printing/sticking means from a left end side of the apparatus main body 33 in a direction orthogonal to each of the blood collection tube storage boxes 1 and reaches a right end side of the apparatus main body 33.

Further, a display placing portion H is provided to a front plate 33b on a front side of the apparatus main body 33. The display placing portion H is formed into an inclined surface so that information displayed on a display is easy to see for an operator standing in front of the apparatus. On the display placing portion H, two kinds of displays, a touch panel display 37 and a setting state display portion 38, are provided. The touch panel display 37 functions both as an input portion and a monitor for a built-in board computer (CPU), and has about an 8-inch size. The display 37 includes, for example, an initial operation screen, a main screen, a detailed status screen, and a maintenance menu screen. A logo and a menu are displayed on the initial operation screen. The menu includes operation start, maintenance, shutdown, restart, program termination, etc. On the main screen, there are displayed function keys, a state indication of whether each of the storage boxes 1 is normal or abnormal, and an indication of whether a printer engine of each of a main printer and a separate-type versatile printer is normal or abnormal. For example, the function keys include six keys, i.e., an F1 key to an F6 key. The F1 key is set to function as a stop/restart button, the F2 key is set to function as a reset button, and the F3 key to the F6 key are set to function as switch buttons for switching to each of the screens.

The advanced settings screen includes a screen allowing selection from setting of the printer engines, setting of the storage boxes 1, setting of a label format, and setting of an upper interface. When the setting of the printer engines is selected, it is possible to perform setting of the printer engine of the main printer and the printer engine of the built-in versatile printer. Setting items for the printer engines include setting of a printer engine number, selection of status (active/inactive) of each engine, selection of status (active/inactive) of a barcode scanner, selection of status (active/inactive) of a tube presence check sensor, setting of a label sticking position, etc. Setting items for the storage boxes include setting of a storage-box number, setting of a storage-box display name, selection of status (active/inactive) of each storage box, setting of a tube length, setting of a label sticking position, etc. In the setting of the label format, it is possible to set arrangement of items to be printed on a label. The items to be printed on a label include a kind of test such as biochemical test, immunological test, blood glucose test, hematological test, or clotting test, a patient name, a container name of a blood collection tube, a barcode, and a blood collection volume. In the setting of the upper interface, it is possible to set whether a serial connection or a socket connection is used, and to set a transmission control protocol/internet protocol (TCP/IP) communications protocol.

The setting state display portion 38 displays a setting state of each component of the blood collection tube preparing apparatus, or displays guidance for changing the settings. For changing the settings, for example, the setting state display portion 38 displays the setting state while interlocking with operation buttons of the blood collection tube preparing apparatus itself. Examples of the operation buttons include a restart button, a feed button, and function key buttons such as the F1 to F3 keys. The setting state to be displayed includes states of during idling, waiting for initial communication command, already having received printing command, during maintenance mode, cover open, label shortage detection, head open detection, mount-sheet pressing roller open detection, sheet feeding motor error, pushdown motor error, positioning motor error, side positioning motor error, tube presence check error, empty storage box, stopper error, conveyor conveying time-out, conveyor jam detection, blood collection tube drop error, etc.

Further, as illustrated in the front view of FIG. 2, a tray disposing portion E is provided on a front side of the placing portion C for the printing/sticking means. A blood collection tube, to which the label having the printed patient information, blood collecting test information, and the like has been stuck, is received into a tray 30 prepared in the tray disposing portion E for each patient. In the tray disposing portion E, optical sensors 36a and 36b (see FIG. 1) for detecting presence/absence of the tray 30 are arranged. As the sensors, there is illustrated a type in which the photodetector 36b detects light radiated from the projector 36a. However, the sensors may be a reflection type.

Still further, a board computer placing portion F is provided below the storage box mounting portion B. Control software for controlling the entire apparatus is installed on the board computer (CPU), and the board computer performs control of the blood collection tube storage boxes 1 and control of a label sticking device 7 and a printing device 25. Further, the board computer sends/receives information to/from the HIS or the LIS serving as the upper system, and information from the upper system can be input to the board computer. Further, the board computer is remote controlled by equipment on the upper system side or a doctor's or laboratory technician's LAN-connected PC. For example, upon termination of a blood collection operation, a power source of the board computer and a power source of the blood collection tube preparing apparatus may be shut off by a PC on a blood collection operation administrator side in the LIS, and the power source of the board computer itself and the power source of the blood collection tube preparing apparatus may be simultaneously shut off with an input screen of the board computer displayed on the screen of the touch panel display 37.

As illustrated in the back view of FIG. 3, on a back side of the apparatus main body 33, as an interface with the board computer, there are provided insertion slots S1 to S3, an R232C terminal R, and audio input/output terminals V1/V2 for a radio board or an external connection equipment board, and USB ports U1 to U4, LAN connection ports L1 to L3, a power supply terminal X, and the like used for connecting to a Fax or another external equipment.

In addition, as illustrated in FIG. 4, an opening portion G which faces the placing portion D for the conveying belt 20 is provided on a left side plate 33a of the apparatus main body 33, and the opening portion G is normally closed by an opening/closing door 34. Through the opening portion G, the blood collection tube is manually put onto the conveying belt 20. In this way, through the opening portion G, a rarely-used blood collection tube or a special-size blood collection tube can be individually supplied to the printing/sticking means.

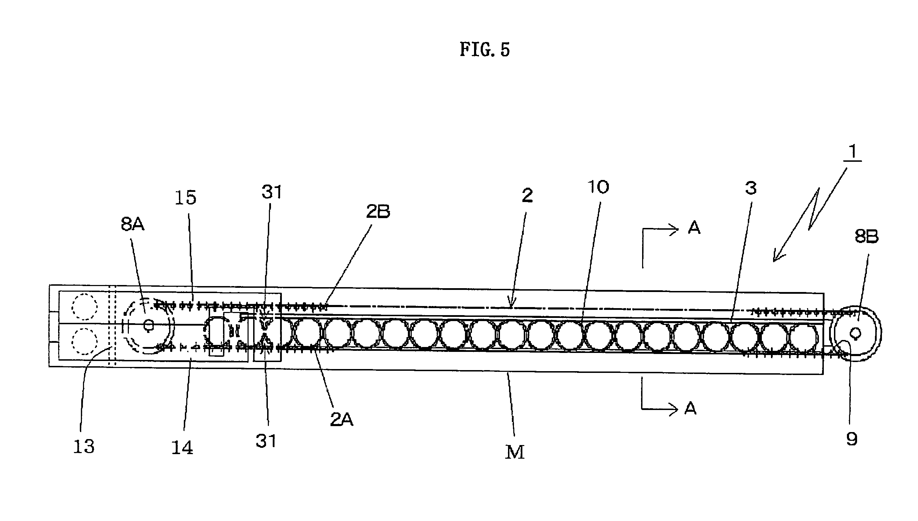

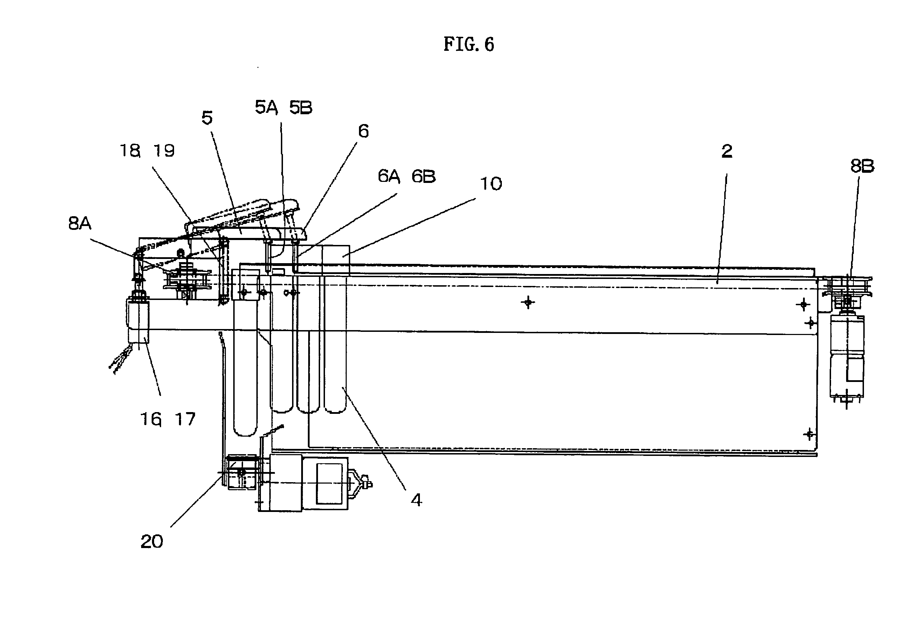

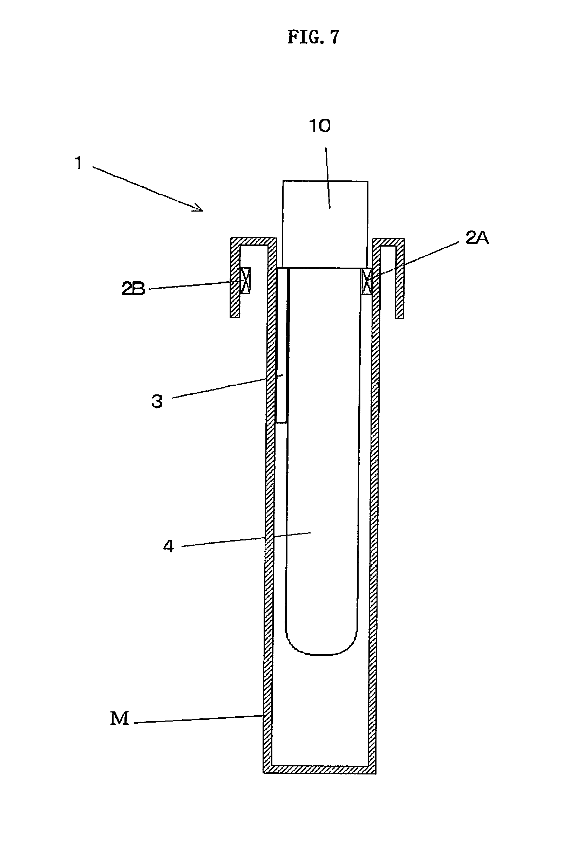

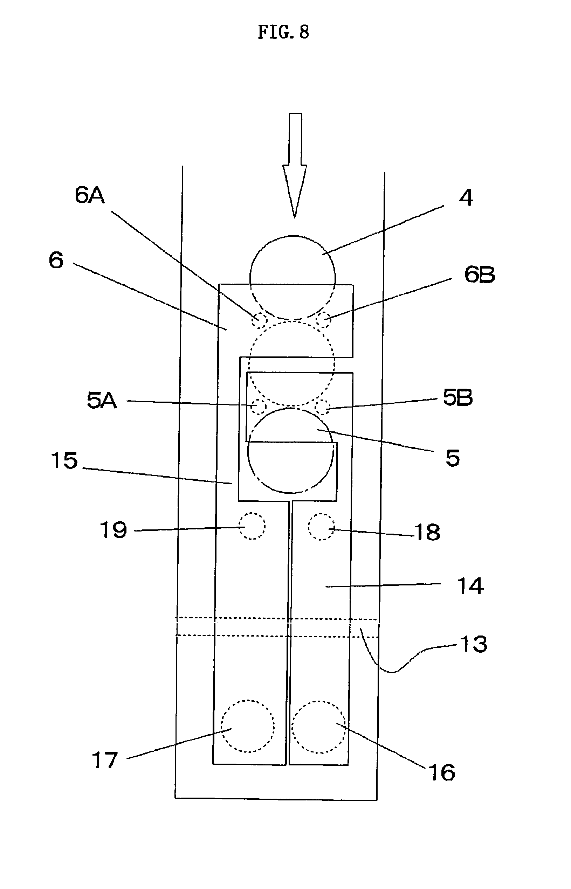

Next, each equipment and mechanism constituting the blood collection tube preparing apparatus A is individually described. First, each of the blood collection tube storage boxes 1 is described with reference to FIGS. 5 to 8. FIG. 5 is a plan view of the storage box 1, FIG. 6 is a side view thereof, FIG. 7 is a vertical sectional view thereof, and FIG. 8 is an enlarged plan view of stoppers. As illustrated in the vertical sectional view of FIG. 7, the blood collection tube storage box 1 in this embodiment includes a casing in which a blood collection tube conveying path (groove) having a U shape in cross-section is formed by bending a thin metal plate made of stainless steel, aluminum alloy, steel, or the like. Each component is coupled to the casing, to thereby structure the entire blood collection tube storage box 1 as one module M. Therefore, the blood collection tube storage box 1 can replaced and repaired in the module M unit through one-touch operation, and maintenance, replacement, and repair operations can be performed at remote locations through sending back only the module M for the operations. In addition, when substitutes for the module M are prepared in advance, it is unnecessary to stop a blood collection operation. Further, through changing a width dimension of the path (groove), it is possible to correspond to sizes (diameters) of blood collection tubes. When the module M is prepared for each of the sizes thereof, it is possible to promptly correspond to various kinds of blood collection tubes.

As illustrated in the plan view of FIG. 5, in the blood collection tube storage box 1, an endless transferring belt 2 is looped around a driven pulley 8A and a drive pulley 8B which are arranged in a laid posture at both ends of the storage box, respectively. The transferring belt 2 is a so-called timing belt having a large number of protrusions 9 provided on an inner surface side on which a forward-side transferring belt 2A and a backward-side transferring belt 2B are opposed to each other. Using the timing belt eliminates slippage between the timing belt and the drive pulley 8B, and enables a drive force of the drive pulley 8B to be reliably transmitted to the transferring belt 2. Note that, as a matter of course, the transferring belt 2 may be a flat belt.

In a space of the transferring belt 2 between the forward-side transferring belt 2A and the backward-side transferring belt 2B, a blood collection tube holding member 3 arranged along the belt is provided. With this structure, a space (groove) for receiving blood collection tubes 4 is formed between the forward-side transferring belt 2A and the blood collection tube holding member 3. Further, the blood collection tube holding member 3 is a guide frame formed of a rail-like or plate-like member or the like having a smoothed upper end surface. The upper end surface is set to be flush with an upper end surface of the transferring belt 2.

Thus, a groove width dimension formed between a distal end surface of each of the protrusions 9 of the forward-side transferring belt 2A and the blood collection tube holding member 3 is set to be equal to or slightly larger (by up to about 1 mm) than an outer diameter dimension of each blood collection tube 4. The groove width dimension can be varied depending on a size of the outer diameter dimension of the blood collection tube 4 to be stored. In order to vary the size, as one method, there may be adopted means for fixing an attachment stay (not shown) for the blood collection tube holding member 3 to a casing main body with a method using an elongated hole and a screw, etc. so as to allow the blood collection tube holding member 3 to move in parallel to the forward-side transferring belt 2A, and thus continuous dimension adjustment is possible. As another method, a module M with a different groove width may be prepared in advance. As the sizes of the blood collection tubes 4, there are generally prepared ones with a diameter of 13 mm and a diameter of 16 mm for biochemical test, immunological test, blood glucose test, hematological test, clotting test, and the like, and ones with a diameter of 8 mm for erythrocyte sedimentation rate test.

As illustrated in FIGS. 6 and 7, each of the blood collection tubes 4 includes a tube-like blood collection tube main body 4A and a cap 10, and a lower end surface of the cap 10 has an outer diameter dimension larger than the outer diameter dimension of the blood collection tube main body 4A (has a dimension larger than the groove width). As illustrated in FIG. 7, each of the blood collection tubes 4 is held by and suspended from the forward-side transferring belt 2A and the blood collection tube holding member 3 in such a manner that opposing lower end surfaces of the cap 10 are placed on the upper end surface of the forward-side transferring belt 2A and the upper end surface of the blood collection tube holding member 3. In other words, the blood collection tubes 4 are stored in a suspended manner. The drive force of the transferring belt 2 is transmitted to the blood collection tubes 4 through a contact portion in the lower end surface of the cap 10 placed on the forward-side transferring belt 2A. The opposite-side lower end surface of the cap 10 only slides with respect to the upper end surface of the blood collection tube holding member 3.

As illustrated in FIG. 7, on a back surface side of the forward-side transferring belt 2A, a member constituting the casing of the module M functions also as a back-up member, and prevents a middle belt from being deflected. As the back-up member for the forward-side transferring belt 2A, there may be used a back-up member structured similarly to the blood collection tube holding member 3 serving as the guide frame. The back surface side of the forward-side transferring belt 2A is supported, and thus the upper end surface of the belt 2A reliably abuts against/is held in contact with the lower end surface of the cap of each of the blood collection tubes 4. With this structure, the forward-side transferring belt 2A is not separated from the lower end surface of the cap 10, and thus the forward-side transferring belt 2A can reliably transfer (feed) the blood collection tubes 4 held in a suspended manner.

On a terminal end side of the blood collection tube holding member 3, a discharging portion 12 for the blood collection tube 4 is provided. The discharging portion 12 is provided in a space formed between the terminal end side of the blood collection tube holding member 3 and the pulley 8A. When one of the blood collection tubes 4 is transferred to the terminal end side of the blood collection tube holding member 3, the lower end surface of the cap 10 is disengaged from the blood collection tube holding member 3 so that the blood collection tube 4 naturally falls down by its own weight.

Incidentally, at a position facing the discharging portion 12, a first stopper 5 and a second stopper 6 are provided. As illustrated best in the plan view of FIG. 8, the first stopper 5 and the second stopper 6 respectively have an arm 14 and an arm 15 which are pivotally and swingably supported by the same shaft 13 at middle portions of the arms, and on a lower surface on one end side of the arm 14/arm 15, fork-like and bar-like protrusions 5A and 5B/protrusions 6A and 6B are provided upright. Of portions of the first stopper 5 and the second stopper 6 provided with the bar-like protrusions 5A and 5B and the protrusions 6A and 6B, portions bent into an L shape in plan view are arranged in a staggered manner so that one bend portion is fitted inside the other bend portion so as not to interfere with each other. The first stopper 5 is capable of moving close to and away from the front surface side of the cap of the leading blood collection tube 4 so as to abut against the same, and the second stopper 6 is capable of moving close to and away from a gap formed between the caps of the leading and second blood collection tubes 4.

Further, a rod 16A of a solenoid 16 and a rod 17A of a solenoid 17 are coupled onto lower surfaces on the other end side of the arms 14 and 15, respectively. As the solenoids 16 and 17 in this case, there are adopted ones having substantially the same outer diameter dimension as that of a ball-point pen for the purpose of downsizing. When the power source is turned ON, the rods 16A and 17A are operated to retract, to thereby swing the arms 14 and 15 to upward positions as illustrated by chain lines of FIG. 6. Note that, the solenoids 16 and 17 are normally set to perform the operation alternately. Return springs (tension springs) 18 and 19 are fixed between the pivot shaft 13 of the respective arms 14 and 15, and the bar-like protrusions 5A and 5B and the protrusions 6A and 6B. This is adopted to return the arms 14 and 15 to horizontal postures due to tensile forces of the return springs 18 and 19 when the power sources of the solenoids 16 and 17 are turned OFF. Further, the return springs 18 and 19 also function to prevent the falling blood collection tubes 4 from coming out to the front side.

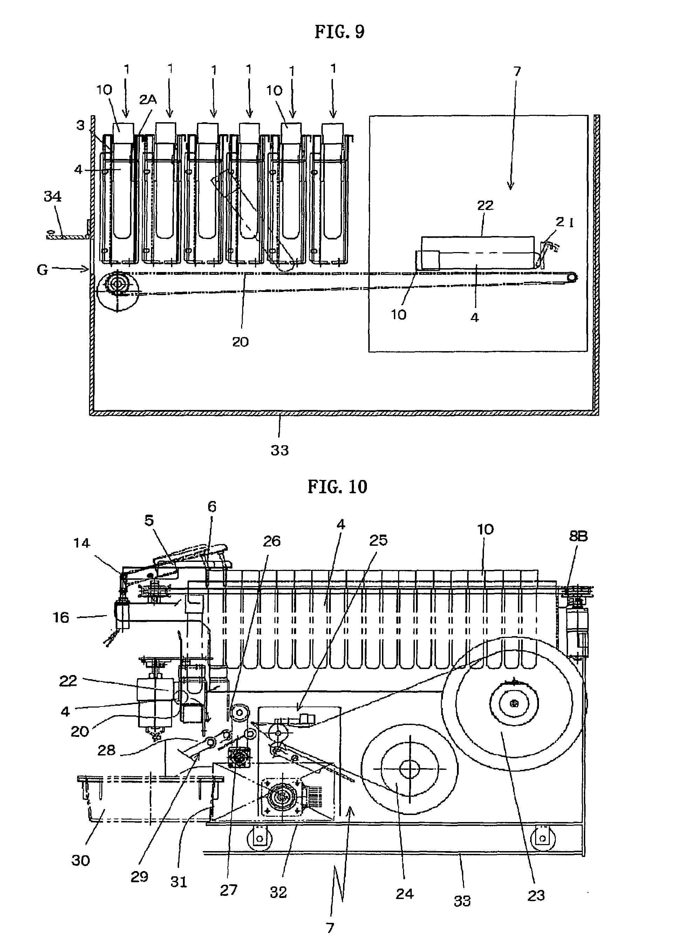

In this embodiment, as illustrated in FIGS. 1 and 9, the blood collection tube storage boxes 1 of a suspended type structured as described above are aligned in six rows. The one conveying belt 20 is provided below the discharging portion 12 in a direction orthogonal to a conveying direction of the transferring belt 2, and each of the blood collection tubes 4 supplied, while falling down, from each of the storage boxes 1 aligned in the six rows is conveyed by the conveying belt 20 from a left side of FIGS. 1 and 9 to a right side thereof. At this time, the lower end surface of the cap 10 of each of the blood collection tubes 4 is disengaged from the blood collection tube holding member 3 on the terminal end side, and hence the cap 10 is inclined to a side disengaged from the blood collection tube holding member 3. In this way, as illustrated by chain lines of FIG. 9, a bottom side of each of the blood collection tubes 4 is always directed forward in the conveying direction, and each of the blood collection tubes 4 is supplied, while falling down in this state, onto the conveying belt 20.

Note that, the conveying belt 20 is constituted by one continuous belt in the embodiment of FIG. 9. However, it is possible that two conveying belts are separately provided in a region for the storage boxes 1 and a region for the label printing/sticking means 7 to allow each of the blood collection tubes 4 to be automatically transferred from one conveying belt to the other conveying belt. In this case, the blood collection tube preparing apparatus has a structure in which a storage box device and a label printing/sticking device are separately provided, and thus the storage box device can be used independently. Further, maintenance can be performed under a state in which the devices are separated from each other, which is convenient.

An arrival detecting plate 21 for the blood collection tubes 4 is provided on the terminal end side of the conveying belt 20. The arrival detecting plate 21, in a normal state, is on standby in an obliquely inclined posture as illustrated by chain lines of FIG. 9. When the blood collection tube 4 is conveyed, the arrival detecting plate 21 is pressed by the blood collection tube 4 to assume an upright posture as illustrated by solid lines, and simultaneously determines a stop position of the blood collection tube 4. The arrival detecting plate 21 has a portion bent into a C shape. When the arrival detecting plate 21 assumes the inclined standby state, light radiated from a detection sensor (not shown) including a projector and a photodetector, such as an infrared ray sensor or a photosensor, is blocked, and when the blood collection tube 4 has arrived so that the arrival detecting plate 21 assumes the upright state, the light radiated from the projector is received by the photodetector. In addition, as illustrated in FIGS. 9 and 10, at a side surface portion of the blood collection tube 4 at the stop position, there is provided a slider 22 for isolating the blood collection tube 4 from the other blood collection tubes, which reciprocates in a direction orthogonal to the conveying direction of the conveying belt 20 and pushes out the blood collection tube 4 from the conveying belt 20, to thereby supply the blood collection tube 4 to a sticking position for the label sticking device 7 while the blood collection tube 4 falls down.

Note that, orientation detecting means (not shown) for detecting an orientation of the blood collection tube 4 is provided just before the arrival detecting plate 21. This is structured to be able to detect the orientation of the blood collection tube 4 in the case where the cap 10 blocks the light radiated from the projector when the cap 10 improperly abuts against the arrival detecting plate 21 first so that the blood collection tube 4 is stopped.

As illustrated in FIG. 10, the label sticking device 7 includes a feeding roll 23 for feeding a label adhering onto a continuous sheet of release paper and a winding roll 24 for winding the sheet of release paper. The label and the sheet of release paper fed from the feeding roll 23 are moved while being folded back at an acute angle so that only the stiff label (which is more elastic and less foldable than the sheet of release paper) is peeled off from the sheet of release paper. The label is moved straight ahead as it is to be fed to a side of sticking means 29, and only the remaining sheet of release paper is wound by the winding roll 24. The printing device 25 for printing patient information and the like onto the label is provided on an upstream side of the releasing portion. The information to be printed is obtained from a database or a hard disk of a computer such as an upper medical system or a doctor's computer terminal by connecting to the computer or the computer terminal through an interface of the board computer (CPU) having a built-in control portion of the blood collection tube preparing apparatus. In addition, in an advancing direction of the label peeled off from the sheet of release paper, the sticking means 29 including a drive roller 26, a support roller 27, and a pressure roller 28 capable of advancing/retreating is provided. Further, a disposing portion for the tray 30 is provided obliquely below the sticking means 29.

Further, as illustrated in FIGS. 1 to 3 and 10, a built-in versatile printer 31 is provided at a position facing the tray 30 and being next to the label sticking device 7 and the printing device 25. The versatile printer 31 is used, for example, to print a blood collection instruction and thereafter to put the instruction onto the tray 30. Further, in a hospital ward or the like, in the case where it is uncertain whether or not a patient to be subjected to blood collection is in a condition of allowing blood collection, only printing of necessary information, such as patient information and test information, on the label is performed by the separate-type printer 31 without sticking the label onto the blood collection tube, and then the label is delivered onto the tray 30. With this structure, it is possible to perform blood collection directly by the patient's bedside depending on the condition of the patient, and to stick the manual sticking label onto the blood collection tube after performing the blood collection. When the patient is in a condition of not allowing blood collection, the manual sticking label may be left in the tray 30.

In addition, as illustrated in FIGS. 3 and 10, the label sticking device 7, the printing device 25, and the versatile printer 31 are mounted on a slide table 32 as a unit, and can be pulled out to the outside of the apparatus main body 33 through detaching an electrical connection mechanism (coupler), a positioning lock mechanism, and the like. Thus, in the case where the label sticking device 7, the printing device 25, or the separate-type printer 31 fails, prompt repair can be made advantageously only through replacing the failed equipment. Commercially available general-purpose products can be used as the label sticking device 7, the printing device 25, and the separate-type printer 31, which is advantageous in terms of low-cost supply. Further, maintenance can be performed on the equipment under a state in which the equipment is pulled to the outside of the apparatus main body 33, and hence the maintenance operation is facilitated.

Next, operations of the blood collection tube preparing apparatus A structured as described above are described. First, the modules M of the blood collection tube storage boxes 1 for storing different kinds of blood collection tubes are set on six-row mounting portions in the storage box placing portion B provided on the upper surface of the apparatus main body 33. The modules M may be fixed with a screw fixing method or another fixing method such as one-touch clip method. Then, the blood collection tubes 4 are set manually in the groove (portion for storing the blood collection tubes 4) formed between the blood collection tube holding member 3 and the forward-side transferring belt 2A of each of the blood collection tube storage boxes 1, and another kind of blood collection tubes 4 are also set manually in another groove. When setting each of the blood collection tubes 4, the blood collection tube 4 only needs to be inserted into the above-mentioned groove from the bottom side thereof. In this way, the opposing lower end surfaces of the cap 10 of each of the blood collection tubes 4 are placed in a bridge manner on the upper end surface of the blood collection tube holding member 3 and the upper end surface of the forward-side transferring belt 2A to be held thereon in a suspended manner. In a state before starting the operation, the bar-like protrusions 5A and 5B of the first stopper 5 of the blood collection tube preparing apparatus abut against the front surface side of the cap 10 of the leading blood collection tube 4, to thereby restrict the forward movement of the leading blood collection tube. Further, the bar-like protrusions 6A and 6B of the second stopper 6 enter gaps 31 and 31 formed between both right- and left-side surfaces of the cap 10 of the leading blood collection tube 4 and the cap 10 of the second leading blood collection tube 4, to thereby restrict the forward movement of the second leading blood collection tube 4.

The power source switch of the apparatus main body 33 is turned ON under this state, and the CPU of the built-in board computer is operated to cause the control software to function. As a result, blood collection information for the patient to be subjected to blood collection is obtained from the upper computer or the doctor's computer terminal. Note that, the control portion (firmware) of the blood collection tube preparing apparatus A does not allow each equipment to operate under a state in which another tray 30 is not newly prepared for the patient after detecting that the tray 30 for the previous patient is removed from the tray disposing portion E. This is intended to prevent confusion which may occur when the blood collection tube for next patient is put onto the tray 30 for the previous patient.

The control portion determines, based on the obtained information, a kind of the blood collection tube to be selected, and drives the drive pulley 8B of the blood collection tube storage box 1 storing the corresponding kind of the blood collection tubes 4 to rotate. The setting information and the information obtained from the upper system are displayed on the touch panel display 37. Owing to drive of the drive pulley 8B, in all of the blood collection tubes 4 of the storage box 1, the forward-side transferring belt 2A applies a transferring force to the lower end surfaces of the caps 10 held in contact with the upper end surface of the forward-side transferring belt 2A. Note that, under this state, the first stopper 5 and the second stopper 6 function to restrict the movement of the leading blood collection tube 4 and the second blood collection tube 4, and hence all of the blood collection tubes 4 remain stopped.

Next, the control portion of the blood collection tube preparing apparatus A turns ON the solenoid 16 of the first stopper 5 of the corresponding storage box 1. Thus, the rod of the solenoid 16 is operated to retract, to thereby cause the arm 14 to pivot on the pivot shaft 13 in a counterclockwise direction of FIG. 10. Accordingly, the bar-like protrusions 5A and 5B fixed on one end side of the arm 14 are moved away from the front surface side of the cap 10 of the leading blood collection tube 4, and cancel restriction of the leading blood collection tube 4. The leading blood collection tube 4 assumes a free state, and starts to advance from the stop position after being applied with the transferring force of the forward-side transferring belt 2A. After a while, the leading blood collection tube 4 reaches the discharging portion 12 on the terminal end side of the blood collection tube holding member 3 to be disengaged from the blood collection tube holding member 3, and hence the cap 10 assumes a posture of inclined toward the disengaged blood collection tube holding member 3 so that the blood collection tube 4 is cantilever-supported by the forward-side transferring belt 2A. Finally, the blood collection tube 4 is disengaged also from the forward-side transferring belt 2A, and, as illustrated by chain lines of FIG. 9, falls down onto the conveying belt 20 by its own weight in a state in which the bottom side of the blood collection tube 4 is directed to the advancing direction of the conveying belt 20.

Meanwhile, in the first stopper 5, when the solenoid 16 is turned OFF, the arm 14 is returned to an original horizontal state by the return spring 18. Then, the solenoid 17 of the second stopper 6 is turned ON, to thereby cause the arm 15 to pivot in a counterclockwise direction up to a position illustrated by chain lines of FIG. 10. Thus, the bar-like protrusions 6A and 6B cancel the contact with the cap 10 of the second blood collection tube 4. Accordingly, all of the second and subsequent blood collection tubes 4 advance while receiving the drive force of the forward-side transferring belt 2A, and the front surface side of the cap 10 of the blood collection tube 4, which is moved to be newly situated at the leading edge of the blood collection tubes 4, abuts against the bar-like protrusions 5A and 5B of the first stopper 5 so that the blood collection tube 4 is stopped. Finally, when the solenoid 17 of the second stopper 6 is turned OFF, the arm 15 is returned to an original horizontal state by the return spring 19, and the protrusions 6A and 6B then enter gaps formed between the caps 10 of the blood collection tubes 4 which are moved to be newly situated at the leading edge and the second position of the blood collection tubes 4. Thus, the blood collection tube preparing apparatus A is on standby in this state until next supply instruction of the blood collection tube 4 is issued.

The conveying belt 20 starts to be driven at the same timing of turning ON the solenoid 16 of the first stopper 5. Thus, the blood collection tube 4 supplied while falling down by its own weight is conveyed by the conveying belt 20 from a leftward direction of FIG. 9 to a rightward direction thereof, to thereby press and urge the arrival detecting plate 21. Note that, even when the blood collection tube 4 is supplied onto the conveying belt 20 while falling down and assuming the upright state, the conveying belt 20 is driven from the leftward direction of FIG. 9 to the rightward direction thereof, and hence the blood collection tube 4 loses the balance so that the bottom side of the blood collection tube is conveyed first. The bottom of the blood collection tube 4 on the conveying belt 20 abuts against the arrival detecting plate 21 on the terminal end side of a conveying path so as to press the arrival detecting plate 21, and the blood collection tube 4 is stopped after swinging the arrival detecting plate 21 from the inclined posture to the upright posture.
Note that, at the stop position, an orientation detecting sensor (not shown) for the blood collection tube 4 detects the cap 10 of the blood collection tube 4. In the case of detecting the cap, it is detected that the cap is conveyed first so that the orientation of the blood collection tube is opposite, and hence an operator is informed of this circumstance through a warning issued by means of sound, a flashing signal of a light emitting device, or the like. Further, the fact that the orientation of the blood collection tube 4 is opposite is displayed as an error on the touch panel display 37. In this case, the operator may continue the operation after taking out the blood collection tube 4 and changing the orientation.

The arrival detecting plate 21 is pressed and urged to the upright posture, and thus arrival detecting means such as a light emitting/receiving sensor functions. As a result, it is possible to detect that the blood collection tube 4 has been supplied. In response to the arrival detecting signal, the slider 22, which reciprocates in the direction orthogonal to the conveying direction of the conveying belt 20, starts to operate, and pushes out the blood collection tube 4, which have been arrived, in a lateral direction so that the blood collection tube 4 is supplied, while falling down, to the sticking means 29 side of the label sticking device 7. In the sticking means 29, the pressure roller 28 is at a retreat position, and, when receiving the blood collection tube 4, the pressure roller 28 advances to press and urge the blood collection tube 4 toward the drive roller 26 and the support roller 27. Then, the drive force of the drive roller 26 is transmitted to the blood collection tube 4 so that the blood collection tube 4 is rotated in a circumferential direction.

Meanwhile, in the label printing device 25, necessary items including blood collection information, such as a patient ID, a test item, a kind of blood collection tube, and a blood collection volume, obtained from the upper computer, a doctor's computer terminal, or the like are printed on the label fed from the feeding roller 23. The contents to be printed are displayed on the touch panel display 37, and hence an operator can confirm the contents. As illustrated in FIG. 10, the sheet of release paper, to which the printed label adheres, is moved while being folded back at an acute angle, and thus only the stiff label is moved straight ahead at it is, to thereby be peeled off from the sheet of release paper naturally. Then, the peeled label enters between the drive roller 26 and an outer peripheral surface of the blood collection tube 4, and is wound around and stuck onto the outer peripheral surface of the blood collection tube 4. When the pressure roller 28 is returned so as to retreat obliquely downward (to a left lower side of FIG. 10), the blood collection tube 4 with the label stuck thereon is released from the sticking portion, and falls down by its own weight to be received in the tray 30.

With the above-mentioned procedure, preparation for one blood collection tube of one tube kind is completed. Thereafter, if another blood collection tube is required, the storage box 1 corresponding to the kind of the another blood collection tube is driven, and the above-mentioned operations are sequentially repeated. Thus, the blood collection tube 4 is received in the tray 30. Further, if there is a blood collection tube to be manually supplied, the blood collection tube may be individually supplied onto the conveying belt 20 through a manual-supply opening portion formed in a left side surface of the apparatus main body, and be received in the tray 30 after being subjected to label sticking. In this way, when preparation is completed for blood collection tubes of all tube kinds required based on the information obtained from the upper computer, the doctor's computer terminal, or the like, preparation is completed for blood collection tubes used to perform blood collection on one patient. Thereafter, blood collection tubes for another patient may be prepared in the similar way.

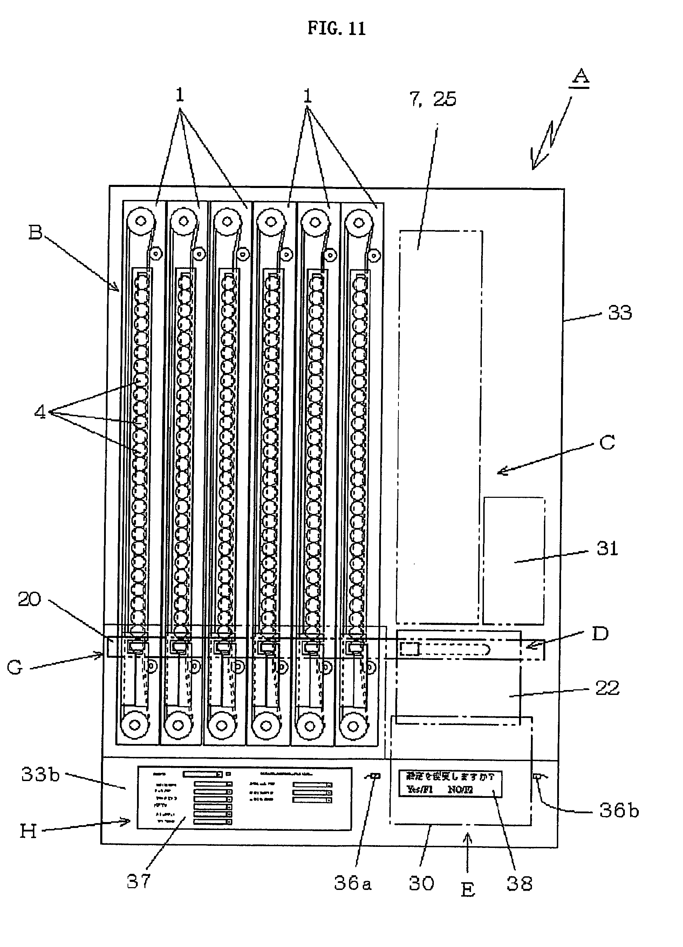

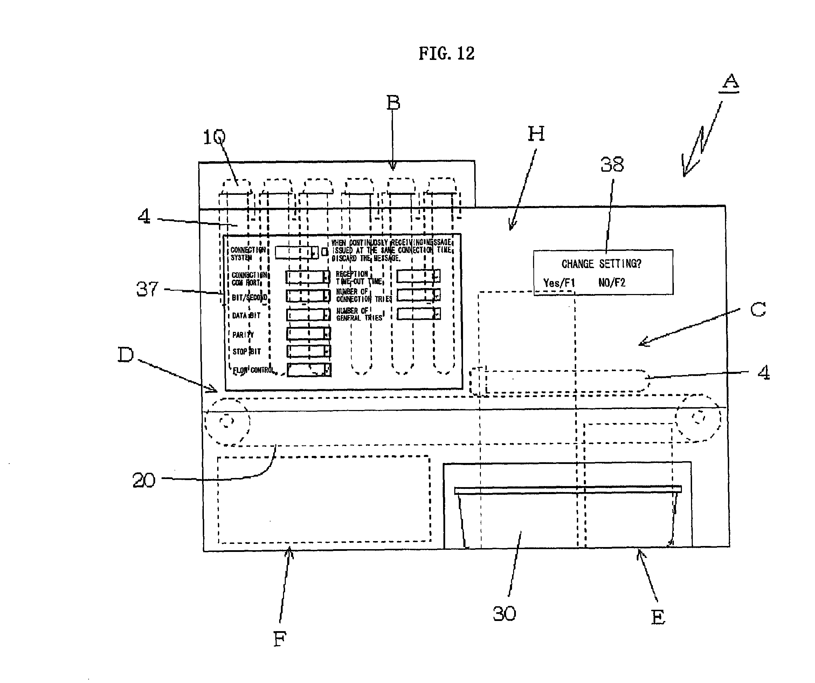

Next, a second embodiment of the present invention is described with reference to FIGS. 11 to 16. FIGS. 11 and 12 illustrate an entire structure of the blood collection tube preparing apparatus A. FIG. 1 is a plan view, and FIG. 2 is a front view. As illustrated in the plan view of FIG. 1, basic layout and structure of the blood collection tube preparing apparatus A are the same as those of the first embodiment. The significant difference from the blood collection tube storage boxes and the blood collection tube preparing apparatus according to the first embodiment is that the blood collection tubes 4 are supported by two support rails 3A and 3B and the transferring force with respect to the blood collection tubes 4 is applied in such a method that outer peripheral side surfaces of the blood collection tubes 4 are held in surface-contact with the transferring belt. In the following, the description of basically the same structure is omitted, and the difference is described.

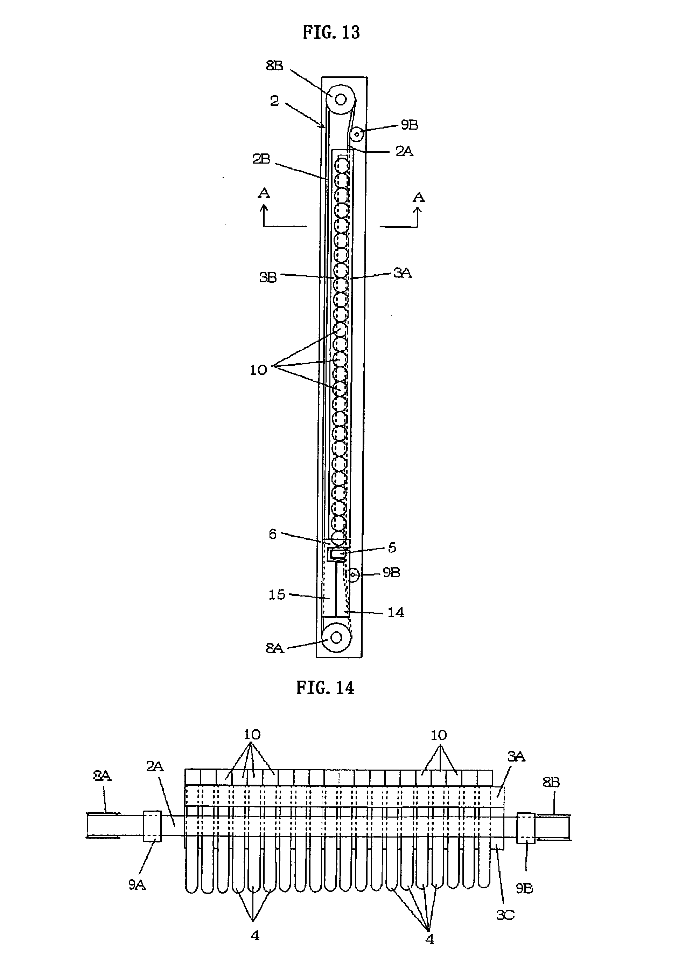

The support rails 3A and 3B are arranged above the upper end of the transferring belt 2 of the blood collection tube storage box 1. The support rails 3A and 3B are coupled to each other on a start side of the support rails (on an upper side of FIG. 13), whereas the support rail 3A is extended so as to be slightly longer than the support rail 3B on a terminal side of the support rails. The support rail 3A is situated just above or slightly outwardly of the forward-side transferring belt 2A, whereas the support rail 3B is situated between the forward-side transferring belt 2A and the backward-side transferring belt 2B. Further, an interval between the support rail 3A and the support rail 3B is set to be slightly larger than the outer diameter dimension of the blood collection tube 4 and smaller than the outer diameter dimension of the lower end surface of the cap 10.

In addition, a tension roller 9A is arranged next to the pulley 8A of the forward-side transferring belt 2A, and a tension roller 9B is arranged next to the pulley 8B thereof. The tension rollers 9A and 9B are provided for bringing the forward-side transferring belt 2A into contact with the outer peripheral side surfaces of the blood collection tubes 4 supported between the support rails 3A and 3B. Further, the support rail 3B includes an extended portion 3C extended on a side opposite to the forward-side transferring belt 2A of the blood collection tubes 4 to support the blood collection tubes 4 through abutting against the blood collection tubes 4. The extended portion 3C is extended downward with respect to the lower end of the transferring belt 3. Therefore, when the tension rollers 9A and 9B abut against the side surfaces of the blood collection tubes 4, the extended portion 3C of the support rail 3B supports the blood collection tubes 4 on the side opposite to the tension rollers so as to prevent the blood collection tubes 4 from slipping off. As a result, it is possible to reliably transmit the drive force of the forward-side transferring belt 2A to the blood collection tubes 4.

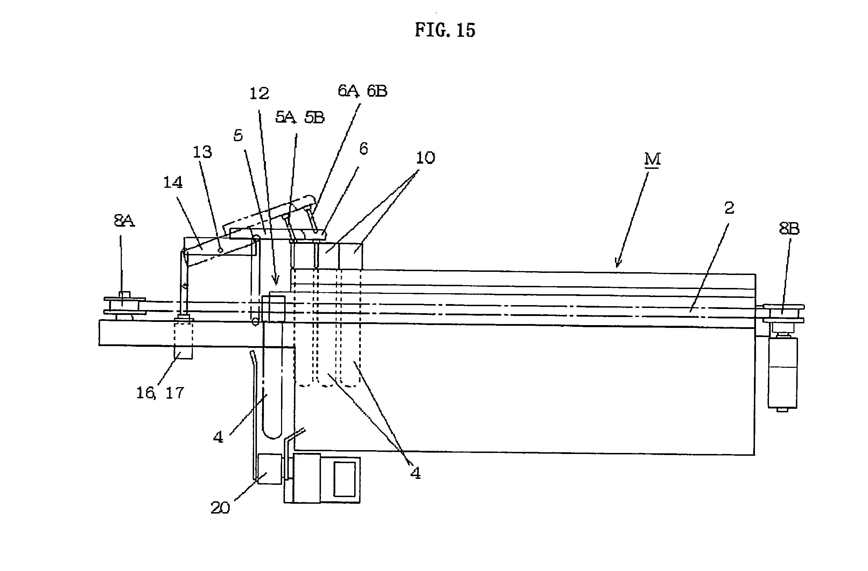

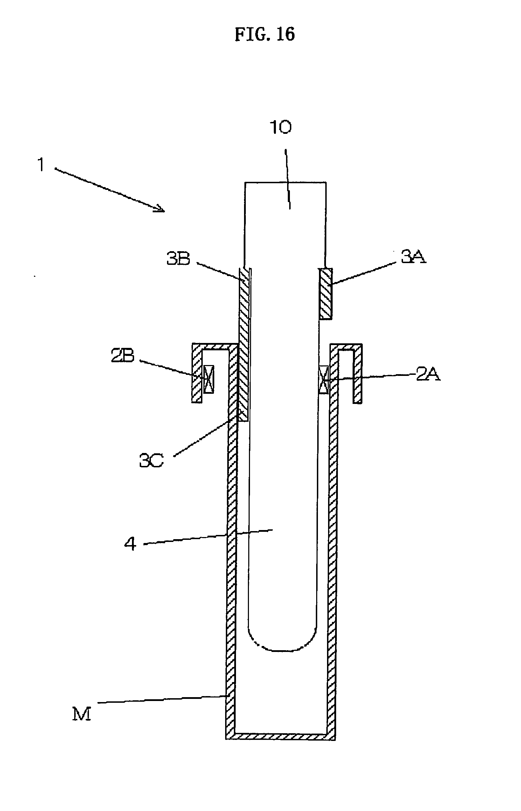

As illustrated in FIGS. 15 and 16, each of the blood collection tubes 4 includes the tube-like blood collection tube main body 4A and the cap 10, and the lower end surface of the cap 10 has the outer diameter dimension larger than the outer diameter dimension of the blood collection tube main body 4A (has the dimension larger than the groove width). As illustrated in FIG. 16, each of the blood collection tubes 4 is held by and suspended from the support rails 3A and 3B in such a manner that the opposing lower end surfaces of the cap 10 are placed on the support rails 3A and 3B. In other words, the blood collection tubes 4 are stored in a suspended manner. The drive force of the transferring belt 2 is transmitted to the blood collection tubes 4 due to a frictional force generated when the tension rollers 9A and 9B bring the forward-side transferring belt 2A into press-contact with the outer peripheral side surfaces of the blood collection tubes 4. The lower end surfaces of the cap 10 only slide on the support rails 3A and 3B.

On a terminal end side of the support rails 3A and 3B, the discharging portion 12 for the blood collection tube 4 is provided (see FIG. 15). The discharging portion 12 is provided in a space formed between the terminal end side of the support rails 3A and 3B and the pulley 8A. The blood collection tubes 4 are discharged in such a manner that, when one of the blood collection tubes 4 reaches the terminal end side of the support rails 3A and 3B, the lower end surfaces of the cap 10 are disengaged from the support rails 3A and 3B so that the blood collection tube 4 naturally falls down by its own weight. One conveying belt 20 is provided below the discharging portion 12 in the direction orthogonal to the conveying direction of the transferring belt 2, and each of the blood collection tubes 4 supplied, while falling down, from each of the storage boxes 1 aligned in the six rows is conveyed by the conveying belt 20 from a left side of FIGS. 11 and 12 to a right side thereof. At this time, the lower end surfaces of the cap 10 of each of the blood collection tubes 4 are disengaged from the support rails 3A and 3B on the terminal end side. Regarding the engagement between the cap 10 and the support rails 3A and 3B, the support rail 3B is set to be shorter than the support rail 3A, and hence the cap 10 is disengaged first from the support rail 3B, and then cantilever-supported by the residual support rail 3A so that each of the blood collection tubes 4 is inclined toward the disengaged support rail 3B. In this way, the bottom side of each of the blood collection tubes 4 is always directed forward in the conveying direction, and each of the blood collection tubes 4 is supplied, while falling down, in this state onto the conveying belt 20.

Note that, the blood collection tube 4 conveyed by the conveying belt 20 is supplied to the label printing/sticking device. When the pressure roller 28 is returned so as to retreat obliquely downward (to a left lower side of FIG. 11), the blood collection tube 4 with the label stuck thereon is released from the sticking portion, and falls down by its own weight to be received in the tray 30. With the above-mentioned procedure, preparation for one blood collection tube of one tube kind is completed. Thereafter, if another blood collection tube is required, the storage box 1 corresponding to the kind of the another blood collection tube is driven, and the above-mentioned operations are sequentially repeated. Thus, the blood collection tube 4 is received in the tray 30. Further, if there is a blood collection tube to be manually supplied, the blood collection tube may be individually supplied onto the conveying belt 20 through the manual-supply opening portion formed in the left side surface of the apparatus main body, and be received in the tray 30 after being subjected to label sticking. In this way, when preparation is completed for blood collection tubes of all tube kinds required based on the information obtained from the upper computer, the doctor's computer terminal, and the like, preparation is completed for blood collection tubes used to perform blood collection on one patient. Thereafter, blood collection tubes for another patient may be prepared in the similar way.

### Industrial Applicability

Incidentally, the present invention is not limited to the above-mentioned embodiments, and may be modified appropriately. For example, description is made on the case where the timing belt is adopted as the transferring belt 2 used for the blood collection tube storage box 1. However, a normal flat belt may be adopted. Further, description is made on the case where six blood collection tube storage boxes 1 are aligned. However, the number of the blood collection tube storage boxes may be below six, for example, one, two, or three, and may be above six. It is possible to arbitrarily set the number of the storage boxes depending on the purpose. The blood collection tube storage boxes 1 can be set onto the apparatus main body 33 in the form of modules, and hence it is possible to easily change the sizes of the blood collection tubes even after shipment. Further, as the modules of the blood collection tube storage boxes 1, for example, a plurality of kinds of modules, such as modules of boxes of ten, fifteen, and twenty tubes, can be prepared so as to store the various numbers of blood collection tubes, and the plurality of kinds of modules can be easily replaced.

Further, description is made on the case where the rods retract when the solenoids 16 and 17 are turned ON. However, the rods may reciprocate, i.e., protrude and retract. In this case, it is possible to omit the return springs 18 and 19.

Still further, it is possible that two conveying belts are provided continuously through partitioning the conveying belt 20 into the region for the storage boxes 1 and the region for the label sticking device 7, to thereby allow each of the blood collection tubes 4 to be automatically transferred from one conveying belt to the other conveying belt. In this case, it is possible to separate external covers constituting the casing of the blood collection tube preparing apparatus into the region for the storage boxes 1 and the region for the label sticking device 7, and hence an individual maintenance operation can be performed in each of the regions. Further, a blood collection tube storage box device and the label sticking device 7 may be structured separately from each other. In this case, it is possible to sell the blood collection tube storage box device alone, and to structure the blood collection tube preparing apparatus through coupling together the blood collection tube storage box device and the label sticking device as an optional product.

Further, the versatile printer 31 is described as a built-in type. However, it is possible that a separately-sold versatile printer is placed at the side of the apparatus A and the separately-sold versatile printer performs printing on a separation-type label through communication connection to the apparatus A. Further, a placement position of the versatile printer is provided next to the main printer 25. However, as long as the versatile printer does not interfere with other equipment in the apparatus main body, the versatile printer may be built in at an arbitrary position. In this case, an eject port for a label to be manually stuck does not need to face a tray feed port.

Still further, it goes without saying that it is possible to appropriately change the information displayed on the touch panel display 37, the assignments and number of function keys, an example of how to display buttons, the contents displayed on the setting state display portion 38, the kind and function of button to be selected, etc. Further, it is possible to appropriately change the placement positions, layouts, and the like of the touch panel display 37 and the setting state display portion 38.

In addition, a groove width dimension formed between the support rails 3A and 3B is set to be equal to or slightly larger (by up to about 1 mm) than the outer diameter dimension of each blood collection tube 4. However, the groove width dimension can be varied depending on a size of the outer diameter dimension of the blood collection tube 4 to be stored. In order to vary the size, as one method, there may be adopted means for fixing an attachment stay (not shown) for the support rail 3B to the casing main body with a method using an elongated hole and a screw, etc. so as to allow the support rail 3B and the extended portion 3C to move in parallel to the support rail 3A. That is, it suffices that continuous dimension adjustment is possible. Further, as another method, a module M with a different groove width may be prepared in advance. As the sizes of the blood collection tubes 4, there are generally prepared ones with a diameter of 13 mm and a diameter of 16 mm for biochemical test, immunological test, blood glucose test, hematological test, clotting test, and the like, and ones with a diameter of 8 mm for erythrocyte sedimentation rate test.

### Reference Signs List

1 ··· blood collection tube storage box
2 ··· transferring belt
2A ··· forward-side transferring belt
2B ··· backward-side transferring belt
3 ··· blood collection tube holding member
3A, 3B ··· blood collection tube support rail
3C ··· extended portion of blood collection tube support rail 3B
4 ··· blood collection tube
5 ··· first stopper
5A, 5B ··· bar-like protrusion
6 ··· second stopper
6A, 6B ··· bar-like protrusion
7 ··· label sticking device
8A, 8B ··· pulley
9 ··· tooth
10 ··· cap
37 ··· touch panel display
38 ··· setting state display portion
A ··· blood collection tube preparing apparatus
B ··· storage box placing portion
C ··· placing portion for printing/sticking means
D ··· placing portion D for conveying belt
E ··· tray disposing portion E
F ··· PC board computer placing portion
G ··· opening portion for manual insertion
M ··· module of blood collection tube storage box

## Claims

1. A blood collection tube storage box, comprising:
an endless transferring belt which is looped around pulleys respectively provided on both end sides of the endless transferring belt, and which is driven to rotate in a horizontal direction;
a blood collection tube holding member which is situated between a forward-side transferring belt and a backward-side transferring belt of the endless transferring belt, and which is arranged along the endless transferring belt,
the forward-side transferring belt and the blood collection tube holding member respectively having upper end surfaces on which opposing lower end surfaces of a cap of a blood collection tube are placed;
a discharging portion through which the blood collection tube falls down by an own weight of the blood collection tube to be discharged, at a position at which the blood collection tube passes a terminal end side of the blood collection tube holding member;
a first stopper and a second stopper provided on the terminal end side of the blood collection tube holding member, the first stopper being capable of moving close to and away from a front surface of a cap of a leading blood collection tube, the second stopper having a fork-like shape and being capable of moving close to and away from a gap from above, the gap being formed between the cap of the leading blood collection tube and a cap of a second blood collection tube;
two arms which are pivotally supported by the same shaft, and to which the first stopper and the second stopper are fixed on distal end sides of the two arms, respectively; and
solenoids coupled on trailing end sides of the two arms, respectively,
the endless transferring belt, the blood collection tube holding member, the discharging portion for the blood collection tube, the first stopper, the second stopper, the two arms, and the solenoids being accommodated in one casing, to thereby form a module.

2. A blood collection tube storage box, comprising:
an endless transferring belt which is looped around pulleys respectively provided on both end sides of the endless transferring belt, and which is driven to rotate in a horizontal direction;
two blood collection tube support rails which are situated between a forward-side transferring belt and a backward-side transferring belt of the endless transferring belt and above an upper end of the endless transferring belt, and which respectively support opposing lower end surfaces of a cap of a blood collection tube in a suspended manner, the forward-side transferring belt having an inner flat surface brought into contact with a side surface of a tube main body of the blood collection tube supported by the two blood collection tube support rails in the suspended manner;
a discharging portion through which the blood collection tube falls down by an own weight of the blood collection tube to be discharged, at a position at which the blood collection tube passes a terminal end side of the two blood collection tube support rails;
a first stopper and a second stopper provided on the terminal end side of the two blood collection tube support rails, the first stopper being capable of moving close to and away from a front surface of a cap of a leading blood collection tube, the second stopper having a fork-like shape and being capable of moving close to and away from a gap from above, the gap being formed between the cap of the leading blood collection tube and a cap of a second blood collection tube;
two arms which are pivotally supported by the same shaft, and to which the first stopper and the second stopper are fixed on distal end sides of the two arms, respectively; and
solenoids coupled on trailing end sides of the two arms, respectively,
the endless transferring belt, the two blood collection tube support rails, the discharging portion for the blood collection tube, the first stopper, the second stopper, the two arms, and the solenoids being accommodated in one casing, to thereby form a module.

3. A blood collection tube preparing apparatus, comprising:
a plurality of blood collection tube storage boxes, each of the plurality of blood collection tube storage boxes comprising:
an endless transferring belt which is looped around pulleys respectively provided on both end sides of the endless transferring belt, and which is driven to rotate in a horizontal direction;
a blood collection tube holding member which is situated between a forward-side transferring belt and a backward-side transferring belt of the endless transferring belt, and which is arranged along the endless transferring belt,
the forward-side transferring belt and the blood collection tube holding member respectively having upper end surfaces on which opposing lower end surfaces of a cap of a blood collection tube are placed;
a discharging portion through which the blood collection tube falls down by an own weight of the blood collection tube to be discharged, at a position at which the blood collection tube passes a terminal end side of the blood collection tube holding member;
a first stopper and a second stopper provided on the terminal end side of the blood collection tube holding member, the first stopper being capable of moving close to and away from a front surface of a cap of a leading blood collection tube, the second stopper having a fork-like shape and being capable of moving close to and away from a gap from above, the gap being formed between the cap of the leading blood collection tube and a cap of a second blood collection tube;
two arms which are pivotally supported by the same shaft, and to which the first stopper and the second stopper are fixed on distal end sides of the two arms, respectively; and
solenoids coupled on trailing end sides of the two arms, respectively,
the endless transferring belt, the blood collection tube holding member, the discharging portion for the blood collection tube, the first stopper, the second stopper, the two arms, and the solenoids being accommodated in a module serving as one casing, the plurality of blood collection tube storage boxes being detachably and respectively received, in a form of the modules, in a plurality of lanes aligned in parallel to each other;
a conveying belt provided below a discharging section ranging over a region of the discharging portion of each of the plurality of blood collection tube storage boxes;
label printing/sticking means provided on a terminal end side of the conveying belt; and
a tray disposing portion provided in a vicinity of a delivery portion of the label printing/sticking means, for receiving the blood collection tube.

4. A blood collection tube preparing apparatus, comprising:
an endless transferring belt which is looped around pulleys respectively provided on both end sides of the endless transferring belt, and which is driven to rotate in a horizontal direction;
two blood collection tube support rails which are situated between a forward-side transferring belt and a backward-side transferring belt of the endless transferring belt and above an upper end of the endless transferring belt, and which respectively support opposing lower end surfaces of a cap of a blood collection tube in a suspended manner, the forward-side transferring belt having an inner flat surface brought into contact with a side surface of a tube main body of the blood collection tube supported by the two blood collection tube support rails in the suspended manner;
a discharging portion which is provided on a terminal end side of the two blood collection tube support rails, and through which the blood collection tube falls down by an own weight of the blood collection tube to be discharged;
a conveying belt provided below the discharging portion, for conveying the blood collection tube;
label printing/sticking means provided on a terminal end side of the conveying belt; and
a tray disposing portion provided in a vicinity of a delivery portion of the label printing/sticking means, for receiving the blood collection tube.

5. A blood collection tube preparing apparatus, comprising:
a plurality of blood collection tube storage boxes, each of the plurality of blood collection tube storage boxes comprising:
an endless transferring belt which is looped around pulleys respectively provided on both end sides of the endless transferring belt, and which is driven to rotate in a horizontal direction;
two blood collection tube support rails which are situated between a forward-side transferring belt and a backward-side transferring belt of the endless transferring belt and above an upper end of the endless transferring belt, and which respectively support opposing lower end surfaces of a cap of a blood collection tube in a suspended manner, the forward-side transferring belt having an inner flat surface brought into contact with a side surface of a tube main body of the blood collection tube supported by the two blood collection tube support rails in the suspended manner;
a discharging portion through which the blood collection tube falls down by an own weight of the blood collection tube to be discharged, at a position at which the blood collection tube passes a terminal end side of the two blood collection tube support rails;
a first stopper and a second stopper provided on the terminal end side of the two blood collection tube support rails, the first stopper being capable of moving close to and away from a front surface of a cap of a leading blood collection tube, the second stopper having a fork-like shape and being capable of moving close to and away from a gap from above, the gap being formed between the cap of the leading blood collection tube and a cap of a second blood collection tube;
two arms which are pivotally supported by the same shaft, and to which the first stopper and the second stopper are fixed on distal end sides of the two arms, respectively; and
solenoids coupled on trailing end sides of the two arms, respectively,
the endless transferring belt, the two blood collection tube support rails, the discharging portion for the blood collection tube, the first stopper, the second stopper, the two arms, and the solenoids being accommodated in a module serving as one casing, the plurality of blood collection tube storage boxes being detachably and respectively received, in a form of the modules, in a plurality of lanes aligned in parallel to each other;
a conveying belt provided below a discharging section ranging over a region of the discharging portion of each of the plurality of blood collection tube storage boxes;
label printing/sticking means provided on a terminal end side of the conveying belt; and
a tray disposing portion provided in a vicinity of a delivery portion of the label printing/sticking means, for receiving the blood collection tube.

6. A blood collection tube preparing apparatus according to any one of claims 3 to 5, further comprising a sensor provided on the tray disposing portion, for detecting presence/absence of a tray,
wherein, when receiving, after receiving the blood collection tube for one patient into the tray, a blood collection tube for a next patient into another tray, each component constituting the blood collection tube preparing apparatus is prevented from starting to operate unless the sensor detects the another tray after detecting that the tray for the one patient is removed.

7. A blood collection tube preparing apparatus according to any one of claims 3 to 6, further comprising an opening portion which is formed in a vicinity of the discharging portion of one of the plurality of blood collection tube storage boxes situated on a most upstream side of the conveying belt, and which allows the blood collection tube to be manually inserted onto the conveying belt through the opening portion.

8. A blood collection tube preparing apparatus according to any one of claims 3 to 7, further comprising a separate-type versatile printer built in at a position in a vicinity of the label printing/sticking means and facing the tray disposing portion.

9. A blood collection tube preparing apparatus according to any one of claims 3 to 8, further comprising a board computer built in a blood collection tube preparing apparatus main body, the board computer being capable of communicating to an upper computer and being provided with various user interfaces.

10. A blood collection tube preparing apparatus according to any one of claims 3 to 9, wherein the label printing/sticking means is slidably attached, as a printer unit, to the blood collection tube preparing apparatus main body so as to be capable of being pulled out of the blood collection tube preparing apparatus main body and replaced by a commercially available product.

11. A blood collection tube preparing apparatus according to any one of claims 3 to 10, further comprising a start switch for the blood collection tube preparing apparatus, which is turned ON and OFF through remote control.

12. A blood collection tube preparing apparatus according to any one of claims 3 to 11, further comprising a setting state display portion provided on an outer surface of the blood collection tube preparing apparatus main body, for displaying a setting state of the blood collection tube preparing apparatus and displaying guidance for changing settings through interlocking with each operation switch.

13. A blood collection tube preparing apparatus according to any one of claims 3 to 12, further comprising:
a central processing unit (CPU) which is built in or externally connected to the blood collection tube preparing apparatus, and on which a software program for controlling the blood collection tube preparing apparatus is installed; and
a touch panel display provided on the outer surface of the blood collection tube preparing apparatus main body, for functioning both as an input portion and a monitor for the CPU.
